# EUROPEAN PATENT APPLICATION

(11) **EP 2 998 409 A1**
(43) Date of publication of application: **23.03.2016**
(21) Application number: 15187228.0
(22) Date of filing: 12.06.2009
(51) Int. Cl.: C12Q 1/70, G01N 33/569, G01N 33/571, C07K 17/08, C07K 16/08

(54) **DETECTION OF LATE STAGES HPV INFECTION**

(30) Priority: 13.06.2008 US 131991 P; 22.09.2008 US 192912 P; 10.06.2009 US 456054; 10.06.2009 US 456053
(62) Divisional of application: 09762928.1
(71) Applicant: OncoHealth Corp., Mountain View, CA 94040 (US)
(72) Inventor: CHENG, Shuling, Fremont, CA 94539 (US)
(74) Representative: Gates, Marie Christina Esther

(57) **Abstract**

Embodiments of the invention provide methods, polyclonal antibodies, monoclonal antibodies, assays, and kits for detecting HPV infection, including infection by various HPV genotypes, early and/or late HPV-associated or HPV-specific proteins or antibodies. Mononoclonal antibodies are used to detect oncogenic high risk and low risk HPV types in a single assay, which is not limited to assay type or format. Useful tools for specific detection of invasive cervical cancer are provided. Cervical cancer biomarkers are identified and can be used in a detection method for early stage precancerous lesions as well as late stage cancer progression.

## Description

### BACKGROUND OF THE INVENTION.

Infection by human papillomaviruses (HPV) at specific epithelium cells to induce epithelial proliferations plays an important role for cervical carcinogenesis. About 99 percent of confirmed cervical cancer cases are found to be associated with HPV infection with biopsy-confirmed squamous intraepithelial lesions (SIL) or cervical intraepithelial neoplasia (CIN). Approximately 1% of the population has genital warts and 4% of women have cervical precancerous lesions, such as low grade of squamous intraepithelial lesion (LSIL) or high grade of squamous intraepithelial lesion (HSIL). or atypical squamous cells of undetermined significance (ASCUS). It is general thought that persistent infection of human papillomavirus (HPV) is essential for developing precancerous epitheliual lesions. Infection of high-risk types HPV for women with LSIL may or may not progress to HSIL. In fact, remission occurs in majority of LSIL human subjects while some progress to HSIL. Although 99.7% of cervical cancers are HPV positive, integration of viral genome into the host genome is required to facilitate the necessary genes to express for developing into HSIL or cancer. Only one in every 10 women with persistent HPV infection may develop into higher grades of CIN lesions, such as cervical intraepithelial neoplasia (CIN) grade 2 and grade 3 (CIN2, and CIN3, respectively), and a portion of these epitheliual lesion cases may ultimately progress into cervical cancer.

Infections by only about 15 HPV types (out of more than 100 available HPV types) are at high risk of developing into cervical intraepithelial neoplasia (CIN) or cervical cancer. Among them, around 70% of reported cervical cancer cases and 50% of reported CIN 2 and CIN 3 cases are caused by two high risk HPV types, i.e., HPV type-16 and HPV type-18. However, some progressive cervical cancer cases are reported to be infected by low risk HPV types, while infection of some high risk HPV types will never progress into cervical cancer. Infections by these two prevailing high risk HPV types do not correlate with tumor development or cancer progression. It seems important to identify those HPV-infected human subjects that express particular oncogenic proteins rather than just identify HPV infection by high risk types.

Thus, there is a need for detect the expression of HPV-related oncoproteins in clinical samples as these oncoprotiens may be better serve as cervical cancer biomarkers to better predict the risk in developing into high grade of cell lesions or cervical cancer-related diseases. There is also a need to develop anti-HPV antibodies and appropriate HPV immunoassays to detect the presence of invasive cervical cancer and/or HPV-related oncoproteins as cervical cancer biomarkers and predict the risk for malignant transformation of epithelial lesions into cervical cancer.

### SUMMARY OF DRAWINGS

Figure 1A shows the representative image of the squamocarcinoma (SCC) tissue from tissue microarray stained by IHC using an anti-E7 monoclonal antibody.
Figure 1 B shows the representative image of the normal epithelium (15 mm away from the tumor tissue) of the SCC subject from Figure 1 A.
Figure 1C shows the representative image of another SCC sample from tissue microarray stained by IHC using the same anti-E7 monoclonal antibody.
Figure 1D shows the magnified representative image of the tumor cells stained in cytoplasm from Figure 1C.
Figure 2A shows the representative image of the tumor cells of adenocarcinoma (ADC) sample stained by IHC using an anti-E7 monoclonal antibody.
Figure 2B shows the representative image of the corresponding normal epithelium (15 mm away from the tumor) of the ADC sample from Figure 2A.
Figure 2C shows the magnified representative image of the cytoplasm staining of adenocarcinoma tumor cells from Figure 2A.
Figure 3A shows a representative staining image of the dysplasia cells of a CIN3 tissue using a mouse monoclonal anti-HPV E7 antibody in an IHC assay according to another embodiment of the invention.
Figure 3B shows the magnified representative image of the dysplasia epithelium of Figure 2A, indicating specific nuclear staining of the CIN3 dysplasia.
Figure 4A shows a representative staining image of the dysplasia cells of CIN2 tissues using an anti-HPV E6 mouse monoclonal antibody in an IHC assay according to one embodiment of the invention.
Figure 4B shows a representative staining image of the normal epithelium adjacent to the dysplasia tissue of the CIN2 sample of Figure 1A using the same anti-HPV E6 mouse monoclonal antibody an IHC assay.
Figure 4C shows the staining results of dysplasia epithelium of a CIN3 tissue using the same anti-HPV E6 mouse monoclonal antibody as the one in Figure 1A in an IHC assay according to another embodiment of the invention.
Figure 4D shows the staining results of dysplasia epithelium of another CIN3 tissue using the same anti-HPV E6 mouse monoclonal antibody as the one in Figure 1C in an IHC assay according to another embodiment of the invention.
Figure 5A shows the staining results of a clinical sample, diagnosed as ASCUS, in a liquid based solution using an anti-HPV E6 mouse monoclonal antibody in an ICC assay according to one embodiment of the invention.
Figure 5B shows the staining results of the same clinical sample as shown in Figure 5A using an anti-HPV E7 mouse monoclonal antibody in an ICC assay according to another embodiment of the invention.
Figure 6A shows the staining results of a clinical sample, diagnosed as CIN2, in a liquid based solution using an anti-HPV E7 mouse monoclonal antibody in an ICC assay according to one embodiment of the invention.
Figure 6B shows the staining results of another clinical sample, diagnosed as CIN2, in a liquid based solution using an anti-HPV E6 mouse monoclonal antibody in an ICC assay according to another embodiment of the invention.
Figure 7A shows the staining results of a clinical sample, diagnosed as CIN3, in a liquid based solution using an anti-HPV E6 mouse monoclonal antibody in an ICC assay according to another embodiment of the invention.
Figure 7B shows the staining results of another clinical sample, also diagnosed as CIN3, and stained with the same anti-HPV E6 mouse monoclonal antibody as the one used in Figure 7A.
Figure 7C shows another ICC staining image on the same clinical sample as used in Figre 7B and stained with the same anti-HPV E6 mouse monoclonal antibody.
Figure 7D shows another ICC staining image on the same clinical sample as used in Figre 7B and stained with the same anti-HPV E6 mouse monoclonal antibody.
Figure 7E shows another ICC staining results on the same CIN3 sample as used in Figre 7B but stained with an anti-HPV E7 mouse monoclonal antibody according to another embodiment of the invention.
Figure 7F shows another ICC staining image of the same CIN3 sample as shown in Figre 7E and stained with the same anti-HPV E7 mouse monoclonal antibody.
Figure 7G shows the staining results on the same CIN3 sample as shown in Figre 3B but stained with an anti-p16 mouse monoclonal antibody in an ICC assay.
Figure 8 shows the staining results of a clinical sample diagnosed as adenocarcinoma and stained with an anti-HPV E6 mouse monoclonal antibody in an ICC assay according to another embodiment of the invention.
Figure 9A shows the staining results of a clinical sample diagnosed as squamous cell carcinoma (SCC) and stained with an anti-HPV E6 mouse monoclonal antibody in an ICC assay according to another embodiment of the invention.
Figure 9B shows the staining results of the same SCC sample as used in Figure 9A and stained with an anti-HPV E7 mouse monoclonal antibody in an ICC assay according to another embodiment of the invention.
Figure 9C shows the staining results of the same SCC sample as shown in Figure 9A using an anti-p16 mouse monoclonal antibody in an ICC assay according to another embodiment of the invention.
Figure 10A shows the staining results of a clinical sample diagnosed as normal and stained with an anti-HPV E6 mouse monoclonal antibody in an ICC assay according to another embodiment of the invention.
Figure 10B shows the staining results of the same clinical sample as shown in Figure 6A and stained with an anti-HPV E7 mouse monoclonal antibody in an ICC assay according to another embodiment of the invention.
Figure 11 A shows staining of the cytoplasmic portions of another cervical scrape sample with an anti-HPV E6 mouse monoclonal antibody in an ICC assay according to another embodiment of the invention.
Figure 11 B shows staining of the nuclear portions of the same sample as used in Figure 11A with an anti-HPV E7 mouse monoclonal antibody.
Figure 11C shows staining of the cytoplasmic portions of another cervical scrape sample with an anti-HPV E6 mouse monoclonal antibody.
Figure 12A shows the staining results of a clinical sample diagnosed as CIN1 and stained with an anti-HPV E6 mouse monoclonal antibody in an ICC assay according to another embodiment of the invention.
Figure 12B shows another ICC staining image of the same CNI1 sample as shown in Figre 12A using the same anti-HPV E6 mouse monoclonal antibody.
Figure 12C shows another ICC staining image of the same CIN1 sample as shown in Figre 12A using the same anti-HPV E6 mouse monoclonal antibody.
Figure 12D shows the staining results of the same CIN1 sample as shown in Figre 12A but stained with an anti-HPV E7 mouse monoclonal antibody in an ICC assay according to another embodiment of the invention.
Figure 12E shows another ICC staining image of the same CIN1 sample as shown in Figre 12D stained with the same anti-HPV E7 mouse monoclonal antibody.
Figure 12F shows the staining results of the same CIN1 sample as shown in Figre 12A using an anti-p16 mouse monoclonal antibody in an ICC assay according to another embodiment of the invention.
Figure 13A shows the results of a dot blot assay to detect HPV L1 proteins using an anti-HPV L1 mouse monoclonal antibody according to one embodiment of the invention.
Figure 13B show the results of another dot blot to detect HPV L1 proteins using the same mouse monoclonal anti-HPV L1 antibody as used in Figure 1.
Figure 14A shows the results of using the same blot as shown in Figure 13A to detect HPV E6 proteins using an anti-HPV E6 mouse monoclonal antibody according to another embodiment of the invention.
Figure 14B shows the results of using the same dot blot shown in Figure 13B to detect HPV E6 proteins using the same anti-HPV E6 mouse monoclonal antibody as the one used in Figure 14A according to another embodiment of the invention.
Figure 15 shows the results of the same dot blot shown in Figure 13A and Figure 14A botting with an anti-HPV E7 mouse monoclonal antibody to detect HPV E7 proteins according to another embodiment of the invention.
Figure 16 is a graph showing the average flourescent intensity results of an antibody microarray assay performed on cell lysate from 10 cervical scrape samples to detect various HPV proteins and various cellular endogenous proteins according to another embodiment of the invention.
Figure 17 is another graph showing the flourescent intensity results of the antibody microarray assay for each of the 10 cell lysate samples shown in Figure 16 to detect HPV L1 proteins according to another embodiment of the invention.
Figure 18 is another graph showing the flourescent intensity results of the antibody microarray assay for each of the 10 cell lysate samples shown in Figure 16 to detect HPV E7 proteins and cellular p16 INK4a proteins according to another embodiment of the invention.
Figure 19 is another graph showing the flourescent intensity results of the antibody microarray assay for each of the 10 cell lysate samples shown in Figure 16 to detect HPV L1 proteins and cellular p53 proteins according to another embodiment of the invention.
Figure 20 is another graph showing the flourescent intensity results of the antibody microarray assay for each of the 10 cell lysate samples as shown in Figure 16 to detect HPV E7 proteins and cellular phosphate form of Rb proteins according to another embodiment of the invention.
Figure 21 is another graph showing the flourescent intensity results of the antibody microarray assay for sample S2.
Figure 22 is another graph showing the flourescent intensity results of the antibody microarray assay for each of the 10 cell lysate samples shown in Figure 16 to detect cellular p21 WAF1 and p53 proteins according to another embodiment of the invention.
Figure 23 shows the results of an ELISA assay to detect HPV E6, E7 and L1 proteins in human serum samples diagnosed as SCC (squamous cell carcinoma) or HPV positive (by PCR) as compared to a HPV negative serum sample (by PCR) using anti-HPV rabbit polyclonal antibody for coating and detection according to another embodiment of the invention.
Figure 24 shows the results of a sandwich beads assay by FACS to detect recombinant HPV16 L1 protein using an anti-HPV16 L1 mouse monoclonal antibody as coating antibody and an anti-HPV16 L1 rabbit polyclonal antibody as detecting antibody.
Figure 25 shows the results of a sandwich beads assay by FACS to detect recombinant HPV16 E6 protein using an anti-HPV16 E6 mouse monoclonal antibody as coating antibody and an anti-HPV16 E6 rabbit polyclonal antibody as detecting antibody.
Figure 26 shows the results of a sandwich beads assay by FACS to detect recombinant HPV18 E6 protein using an anti-HPV18 E6 mouse monoclonal antibody as coating antibody and an anti-HPV18 E6 rabbit polyclonal antibody as detecting antibody.
Figure 27 shows the results of a sandwich beads assay by FACS to detect recombinant HPV16 E7 protein using an anti-HPV16 E7 mouse monoclonal antibody as coating antibody and an anti-HPV16 E7 rabbit polyclonal antibody as detecting antibody.
Figure 28 shows the results of a sandwich beads assay by FACS to detect recombinant HPV16 E6 protein using an anti-HPV16 E6 mouse monoclonal antibody as detecting antibody and an anti-HPV16 E6 rabbit polyclonal antibody as coating antibody.
Figure 29 shows the results of a sandwich beads assay by FACS to detect recombinant HPV18 E6 protein using an anti-HPV18 E6 mouse monoclonal antibody as detecting antibody and an anti-HPV18 E6 rabbit polyclonal antibody as coating antibody.
Figure 30A shows the results of a one-step lateral flow through assay to detect a HPV L1 recombinant protein (from left to right: 875, 435, 0 ug/ml) using a rabbit anti-HPV L1 polyclonal antibody stripped on membrane, and conjugate with gold particle (as the detection agent).
Figure 30B shows the results of a one-step lateral flow through detecting HPV L1 protein in human serum sample from SCC (squamous cell carcinoma) patient (left) compared to a normal (known HPV negative) serum (right) using the same strips shown in Figure 30A.
Figure 30C shows the results of a one-step lateral flow through detecting HPV E6 recombinant protein (from left to right: 10, 2, 0 ug/ml) using a mouse anti-HPV E6 monoclonal antibody stripped on membrane, and gold conjugate for detection.
Figure 30D shows the results of a one-step lateral flow through detecting HPV L1 protein in human serum sample from SCC (squamous cell carcinoma) partient (1^{st} and 2^{nd} from the left) compared to a normal (known HPV negative) serum (right) using the same strips shown in Figure 30C.
Figure 30E shows the results of a one-step lateral flow through detecting HPV E6 recombinant protein (from left to right: 875, 435, 0 ug/ml) using another mouse anti-HPV E6 monoclonal antibody stripped on membrane, and gold conjugate for detection.
Figure 30F shows the results of a one-step lateral flow through detecting HPV E7 recombinant protein (from left to right: 0, 660, 66, 6.6, 0.66, ug/ml) using a mouse anti-HPV E7 monoclonal antibody stripped on membrane, and gold conjugate for detection.
Figure 30G shows the results of a one-step lateral flow through detecting HPV E7 protein from a known HPV positive human serum sample (left) compared to a known HPV negative serum (right) using the same strips shown in Figure 30F.

### SUMMARY OF INVENTION

Embodiments of the invention provide various immunoassays for *in situ* detection of HPV proteins using various monoclonal antibodies against recombinant HPV proteins such that infection by high risk and/or low risk HPV types can be detected by a single specific monoclonal antibody and/or a general pan antibody. In one embodiment, a method of detecting papillomavirus infection in a human subject includes conducting an immunological assay on a clinical sample of the human subject; and staining of a nuclear portion of a human cell from the clinical sample using one or more monoclonal antibodies capable of binding to HPV early viral proteins. In another embodiment, a method of detecting papillomavirus infection in a human subject include conducting an immunohistochemistry assay on humans cells from a clinical sample of the human subject using one or more antibodies generated against one or more purified recombinant papillomavirus proteins and comparing the staining of the nuclear portion with the staining of the cytoplasmic portion of the human cell from the clinical sample. Another method of detecting papillomavirus infection in a human subject includes conducting an immunohistochemistry assay on a clinical sample of the human subject using two or more antibodies capable of binding to a HPV viral protein selected form the group consisting of E6, E7, L1 proteins, and combinations thereof, and comparing the staining of the human cell by the two or more antibodies, wherein positive staining of the human cell by at least one of the two or more antibodies indicates papillomavirus infection in the human subject.

In one aspect, the invention provides an anti-HPV monoclonal antibody capable of being used in an immunological assay to stain a cytoplasmic portion of a HPV-infected human cell from a human subject with a late disease stage. In another aspect, the invention provides an anti-HPV E7 monoclonal antibody and anti-HPV E6 monoclonal antibody capable of being used in an immunological assay to stain a nuclear portion of a human cell from a human subject to indicate HPV infection at a disease stage. The staining of the nuclear portion of the HPV infected human cell indicates early stage HPV infection, whereas the staining of the cytoplasmic portion of the HPV infected human cell indicates dysplasia progression to a late disease stage by HPV infection. The anti-HPV monoclonal antibody used in the immunological assay may be an anti-HPV E7 monoclonal antibody, anti-HPV E6 monoclonal antibody, anti-HPV L1 monoclonal antibody, and combinations thereof. In another aspect, the invention provides an anti-HPV E7 monoclonal antibody and HPV E7 protein as a biomarker used for detection of cervical cancer progression. In addition, the invention provides methods of performing one or more immunological assays, including immunohistochemistry assays and immunocytological assays. Monoclonal antibodies highly specific for HPV viral proteins are also provided to be used in the HPV-detecting immnological assays. In one embodiment, a nuclear portion of an epithelial tissue sample is stained with the anti-HPV monoclonal antibody to indicate HPV infection in general. In another embodiment, a cytoplasmic portion of an epithelial tissue sample is stained with the anti-HPV monoclonal antibody to indicate dysplasia progression by HPV infection.

In still another aspect, a kit for performing an immunological assays on a clinical sample is provided and includes an anti-HPV monoclonal antibody capable of staining a nuclear portion of a HPV infected human cell from a human subject to indicate HPV infection and capable of staining a cytoplasmic portion of the HPV infected human cell to indicate HPV infection at a late disease stage. In one aspect, a kit for detecting papillomavirus infection in a human subject may include an anti-HPV monoclonal antibody for performing an immunological assays on a clinical sample of the human subject, capable of staining a nuclear portion of one or more human cells from the clinical sample to compare the staining of the nuclear portion with the staining of the cytoplasmic portion of the human cells

The invention also provides HPV immunocytochemistry (ICC) assay, HPV flow cytometry assay, HPV immunohistochmistry (IHC) assay to detect the presence of HPV proteins in cervical cells or cervical tissues. In addition, monoclonal antibodies highly specific for HPV viral proteins are also provided to be used in the HPV ICC or HPV flow cytometry assays. In one embodiment, a method of detecting papillomavirus infection in a human subject is conducted by an immunocytological assay to detect *in situ* one or more papillomavirus proteins from one or more papillomavirus types present in a biological sample on a slide containing a thin layer of human cells, using one or more antibodies to stain the thin layer of human cells. The method includes providing a clinical sample from the human subject, the clinical sample is processed into a mixture of morphologically abnormal and normal human cells, applying the mixture into a thin layer of cells on a slide, and conducting an immunocytochemistry assay using one or more antibodies generated against one or more purified recombinant papillomavirus proteins, such that one or more papillomavirus viral proteins from one or more papillomavirus types present in the clinical sample on the slide containing the mixture of morphologically abnormal and normal human cells is stained *in situ.*

In another embodiment, a method of detecting papillomavirus infection in a human subject includes providing a clinical sample from the human subject and tagging one or more antibodies with an agent, wherein the clinical sample is processed into a liquid-based solution containing a mixture of morphologically abnormal and normal human cells. The mixture of the morphologically abnormal and normal human cells is stained using the one or more antibodies generated against one or more purified recombinant papillomavirus proteins. The method further includes conducting one or more flow cytometry assays to detect each individual cells by separating each cell from the mixture of the morphologically abnormal and normal human cells, and detecting the presence of one or more human papillomavirus viral proteins from one or more papillomavirus types in the mixture of the morphologically abnormal and normal human cells contained in the liquid-based solution from the clinical sample.

In one embodiment, the one or more antibodies are generated against one or more purified recombinant papillomavirus proteins, wherein at least one antibody is capable of recognizing a papillomavirus early protein. In another embodiment, the one or more antibodies are tagged with an agent, and one or more human cells from a biological sample of the human subject are prepared into a liquid-based solution, such that the binding of the one or more antibodies with the one or more papillomavirus proteins from one or more papillomavirus types present in the one or more human cells of the biological sample can be detected by the presence of the agent reacting with the tagged one or more antibodies. In another embodiment, the agent includes a colormetric agent, a fluorescent chromogen, and other agent for later separation and identification of the one or more human cells in one or more flow cytometry assays. In addition, a kit for performing an immunocytological assay is provided. The kit may include an pre-antibody blocking solution, post-antibody blocking solution, an anti-HPV antibody as the primary antibody, an anti-mouse or anti-rabbit immunoglobulins conjugated with HRP or biotin, or other agents as secondary antibody, a solution containing appropriate agents used as substrate for the secondary antibody to be detected.

In still another embodiment, a kit is provided for detecting papillomavirus infection in a human subject and includes an anti-HPV monoclonal antibody capable of binding to a papillomavirus early protein for conducting an immunocytochemistry assay on a clinical sample from the human subject processed into a solution containing a mixture of morphologically abnormal and normal human cells and applied into a thin layer of cells on a slide for staining *in situ* one or more papillomavirus viral proteins from one or more papillomavirus types present in the clinical sample on the slide containing the mixture of morphologically abnormal and normal human cells.

The invention also provides HPV blot membrane assay, protein chip microarray assay, HPV beads assay, HPV lateral flow rapid test, HPV vertical flow-through rapid test, HPV microfluidic rapid test, direct enzyme immunoassay (EIA), enzyme linked imunoabsorbant assays (ELISA) to detect the presence of HPV proteins in a biological sampe, such as cervical cells or cervical tissues. In addition, kits and devices for performing these assays are also provided. In one embodiment, a method is provided to detect one or more papillomavirus proteins and includes providing one anti-HPV antibody capable of binding to the one or more papillomavirus proteins from one or more papillomavirus types and present in a clinical sample, providing a solid surface having coated thereon the anti-HPV antibody or the various proteins present in the cell lysate solution, and processing the clinical sample into a cell lysate solution containing various proteins including the one or more papillomavirus proteins. The method further includes reacting the anti-HPV antibody with the cell lysate solution; to form a complex of the one or more papillomavirus proteins with the anti-HPV antibody on the solid surface and detect the complex on the solid surface to confirm the presence of the one or more papillomavirus proteins present in the clinical sample.

In another embodiment, a method is provided for detecting the presence of one or more papillomavirus proteins from one or more papillomavirus types in a biological sample and includes providing a biological sample processed into a cell lysate solution, providing a first anti-HPV antibody immobilized on a solid surface to react with the cell lysate solution, and reacting the cell lysate solution with a second anti-HPV antibody. Both antibodies are capable of binding to one or more papillomavirus proteins from one or more papillomairus types. The method further inlcudes forming a complex of the one or more papillomavirus proteins with the first and the second anti-HPV antibody on the solid surface, and detecting the formation of the complex on the solid surface for the presence of the one or more papillomavirus proteins in the biological sample.

In addition, a lateral flow through device for detecting the presence of one or more papillomavirus proteins from one or more papillomavirus types in a biological sample is provided according to one embodiment of the invention. The lateral flow through device includes a first solid surface of a strip having a first anti-HPV antibody immobilized on one end of the strip and a second solid surface of a strip having a second anti-HPV antibody on the other end of the strip able to react with a cell lysate solution processed from the biological sample for flowing laterally on the solid surface of the strip to form into a complex with first anti-HPV antibody. In another embodiment, a vertical flow-through rapid test device is provided for detecting the presence of one or more papillomavirus proteins from one or more papillomavirus types in a biological sample and includes a solid surface of a membrane having a first anti-HPV antibody immobilized thereon to react with a cell lysate solution processed from the biological sample, and a second anti-HPV antibody to be added with the cell lysate solution onto the solid surface of the membrane for flowing vertically through the solid surface and forming into a complex with first anti-HPV antibody on the solid surface of the membrane.

In one aspect, the first anti-HPV antibody is generated against one or more first recombinant proteins encoded by one or more first papillomavirus genes such that the first anti-HPV antibody is able to capture the one or more papillomavirus proteins in the cell lysate solution onto the solid surface. In another aspect, the second anti-HPV antibody is generated against the same first recombinant proteins encoded by the same papillomavirus genes such that the second anti-HPV antibody is able to bind and detect the one or more papillomavirus proteins in the cell lysate solution.

**1. HPV recombinant proteins to be used as immunogens for generating antiserum and anti-HPV antibodies, and screening hybridoma cell lines** for **monoclonal antibodies.** HPV recombinant proteins can be any kinds of HPV proteins, HPV proteins of early genes and/or late genes, including, but not limited to, E2, E6, E7, L1, L2 and can be from various HPV types. Full-length E6, E7, and/or L1 polypeptide sequence, which have been found very difficult to obtain and purify due to undesirable aggregation during protein purification, protein instability, low levels of expression, low immunogenic responses of purified proteins. For example, many early E6 oncoproteins contain many cysteine amino acids and thus the correct topography of the E6 oncoproteins requires formation of many disulfide bonds properly. In addition, it was known that certain immunological assays using small peptides of early E6 and E7 proteins results in extremely low assay specificity and sensitivity and thus unsuitable as tools for clinical in vitro diagnostics. Thus, the invention provides recombinant proteins, such as recombinant hybrid proteins containing a partial sequence or a full length sequence of HPV oncogenic proteins.
1). Cloning and production of various recombinant proteins encoded by HPV16 E6 and HPV18 E6 gene. An exemplary oncogenic E6 early gene from an exemplary HPV type, HPV-16, was clone. The HPV 16 E6 gene cloned herein is a 474 base pair (b.p.) DNA fragment containing the 157 amino acid coding region of the whole HPV-16 E6 gene and obtained by polymerase chain reaction (PCR) amplification. The DNA sequence of the isolated DNA fragment was confirmed by comparing with the sequence from Gene Bank database. Recombinant HPV-18 E6 protein was also obtained. All cloning procedures are carried out according to the protocols described in "Molecular Cloning", A Laboratory Manual, eds. Sambrook, Fritsch and Maniatis, Cold Spring Harbor Laboratory Press, 1989. In addition, HPV18 E6 gene was also cloned and the DNA sequence was confirmed.
2). Cloning and production of various recombinant proteins encoded by HPV16 E7 and HPV18 E7 gene. An exemplary oncogenic E7 early gene from an exemplary HPV type, HPV-16, was cloned. A 294 base pair (b.p.) DNA fragment containing the 99 amino acid coding region of the entire HPV-16 E7 gene was obtained by polymerase chain reaction (PCR) amplification. The DNA sequence of the isolated DNA fragment was confirmed by comparing with the sequence from Gene Bank database. Recombinant HPV-18 E7 protein was also obtained In addition, E7 DNA fragments from different HPV types can also be cloned from different clinical samples or sources.
3). Cloning and production of various recombinant proteins encoded by HPV16 L1 and HPV18 L1 gene. An exemplary late gene from an exemplary HPV type, HPV-16, was cloned. A 1596 base pair (b.p.) DNA fragment containing the 531 amino acid coding region of the HPV-16 L1 gene was obtained by polymerase chain reaction (PCR) amplification. The DNA sequence of the isolated DNA fragment was confirmed by comparing with the sequence from Gene Bank database. In addition, L1 DNA fragments from different HPV types can also be cloned from different clinical samples or sources.

A recombinant N-terminal fragment of HPV 16 L1 protein was also obtained from a His-tagged expression system. The molecular weight of the HPV-16 L1 N-terminal recombinant protein ais bout 34 KD. L1 C-terminal fragments can also be obtained. Recombinant HPV-18 L1 protein was also obtained and used as immunogens for generating antiserum, polyclonal and monoclonal antibodies.

The one or more recombinant proteins as described herein can be expressed in various suitable systems, such as bacterial expression systems, viral expression systems, yeast expression systems, mammalian expression systems, e.g., in *E coli,* yeast, baculovirus, and/or mammalian cell cultures, generally known in the field. Although the polypeptides could be obtained by other means, embodiments of the invention provide one or more recombinant proteins mostly in (or close to) their native forms, which may be a much desirable conformation for binding with antibodies from tissues of human subjects with HPV infection in an immunological assay. For example, GST, MBP, or His tagged-HPV16-E6, HPV18 E6, HPV16 E7, HPV18 E7, HPV16 L1, and HPV18 L1 recombinant proteins were expressed in *E.coli BL21(DE3)* using IPTG driven induction. After induction of protein expression, tagged-HPV recombinant proteins were obtained from soluble fraction after lysis of the cultured cells and purified to a final concentration of about 0.1 to 1 mg/ml or higher. The purity of the recombinant HPV proteins was estimated to be>90% based on PAGE analysis. Recombinant HPV proteins were used to detect the presence of HPV antibody in clinical samples and also used as immunogens for producing polyclonal antiserum and monoclonal antibodies.

The cell culture containing various recombinant papillomavirus proteins in various expression vectors as described herein are then scaled up to 1 liter or 10 liter, or 100 liters or higher to obtain high quantity of soluable recombinant protein for purification. The soluble fraction after cell lysis was passed through various chromatograpgy columns with appropriate expression systems to bind to the tag expressed along with the HPV recombinant proteins. The tag-HPV recombinant proteins were then eluted from the column and concentrated down to 100 ml or 10 ml to 1 ml. The purified soluble recombinant HPV proteins were further concentrated and dailysed with buffers at neutral pH or PBS buffers to be used as immunogen to generate antiserum against the HPV proteins. The soluble recombinant HPV proteins were thus purified from soluble fractions and folded close to their native folding states as in vivo natural conditions.

Obtaining high quality purified recombinant HPV proteins is critical in generating various types of monoclonal antibodies that recognizing common epitopes or specific epitopes for detecting HPV infection. The purified recombinant HPV proteins were tested to confirm its binding to the HPV antibody from the HPV infected clinical samples. Thus, such purified recombinant HPV proteins are suitable for use as immunogen to raise antiserum and generate antibodies that can recognize natural HPV viral proteins *in vivo.*

**2. anti-HPV polyclonal antibody production:** Recombinant HPV E6, E7 or L1 proteins expressed in *E coli* was purified, concentrated, and dialyzed with PBS to be used as immunogens. Immunization was followed by standard protocol. Titer of each serum obtained was tested by ELISA assays followed by periodical boosting and bleeding. Production bleed from optimal titer was collected; processed serum was used to do immunoglobulin (Ig) purification via protein A columns or affinity columns. Purified IgG was used as anti-HPV antibodies for HPV immunoassays.

Monoclonal antibodies, polyclonal antibodies, and antiserum were obtained, purified, and tested herein to be able to detect HPV infection regardless of the pathogenesis of HPV infection, cell lesions, inflammatory, or cancer disease development. Other researchers have tried to develop anti-HPV monoclonal antibodies but have failed because they failed to generate sufficient HPV proteins for monoclonal antibodies production; they failed to generate monoclonal antibodies with high specificity because the immunigens were not immunogenic; or the generated antibodies were not able to recognize native forms of HPV proteins present in clinical samples with early stage HPV infection. Some antibodies raised against mutant peptides were only able to recognize late stage cervical cancer, but are not sure whether their antibodies would recognize wild type HPV native proteins or any early stage HPV infection. In addition, late stage HPV detection is too late for disease intervention and treatment.

The clinical utility of the antibodies described herein was validated by HPV immunoassays, such as ELISA assays, immunocytochemistry assays, immunohistochemistry assays, using appropriate clinical samples. The novel monoclonal antibodies and antiserum, obtained from methods of this inventon are able to interact and bind HPV viral proteins present in clinical samples, which have been confirmed to contain early stage cell lesions such as cervical intraepithelial neoplasia (CIN) as well as late stage HPV associated cervical cancer. These monoclonal antibodies provide powerful tools to detect and screen HPV related pathogenesis and cervical cancer development in both early stages and late stages; thus provide an avenue to intervene disease progression and a chance to provide early treatment.

**3. HPV Monoclonal antibody development:** Recombinant HPV E6, E7 or L1 proteins expressed *in E coli* was purified, concentrated, and dialyzed with PBS to be used as immunogen. Immunization of mice was followed by standard procedure. Titer of the obtained serum was tested by ELISA followed by periodical boosting and bleeding. When the titer of the serum of the mice reaches optimal, fusion of the spleen cells of the mice with tumor cells was done by standard procedure. Clones of fused cells, e.g., hybridoma cells, were further cultured.
1). Hybridoma screening: To obtain anti-HPV antibody producing hybridoma cells with pan and speicifc binding capability to various HPV proteins as described in this invention, hybridoma clones were screened with various proteins, including, not only the original immunogens but also additional HPV proteins as positive screening, and unrelated proteins as negative screening. For example, two or more purified HPV recombinant proteins were used to screen against each hybridoma clone to screen and obtain monoclonal antibody-producing hybridoma cell lines and to test and understand the specificity of each antibody-producing hybridoma cell line thus obtained.
2). Hybridoma cell line stocks: Hybridoma cell line clones with desired positive reactivity and desired negative reactivity as judged by an immunosays (e.g., ELISA, EIA and other assays) were selected and cloned down to single cell. Each single cell clone was then grown up by tissue culture. When the cell numbers reach millions of cells per ml, the cells were frozen down and kept at -80 °C or in liquid nitrogen as storage stocks.
3). Ascites production: Each hybridoma cell line was grown in tissue culture and injected to mice for ascites production. Ascites were collected and processed for immunoglobin purification by protein G columns. Purified immunoglobin from each hybridoma cell line was isotyped and used for HPV immunoassays.

**4. HPV Immunohistochemisty (IHC) assay:** In one embodiment, a kit for performing a HPV IHC assay is provided. The kit may include an antigen retrival agent, a pre-antibody blocking solution, a post-antibody blocking solution, an anti-HPV antibody as the primary antibody, an anti-mouse or anti-rabbit immunoglobulins conjugated with HRP or biotin, or other agents as secondary antibody, a solution containing appropriate agents used as substrate for the secondary antibody to be detected.

The antigen retrival agent may contain a solution in low pH, or neutral pH or high pH buffer. The pre-antibody blocking solution may contain certain proteins or BSA, or serum or other agents to block the cells from nonspecific binding of antibody. The post blocking solution may contain similar solution as the pre-antibody blocking solution with less proteins or serum to be used along with primary antibody incubation. The solution containg HPV antibodies may be in concentrated form, or may be in diluted form as ready to use reagent. The anti-HPV antibodies may also be directly tagged with HRP or biotin, or other agents to be detected following appropriate agents used as substrate. The solution containing secondary antibodies may be in concentrated form, or may be in diluted form as ready to use reagent. The solution containing appropriate agents used as substrate may include DAB (3.3'-diaminobenzidine) as one component, or two components, or AEC (3-Amino-9-Ethylcarbazole) substrate as one component, or two components, or other substrates. Once the cervical tissues are processed and fixed, the Immunohistochemistry (IHC) assay is performed by boiling the tissues on the slide with antigen retrival buffer for a period of time. The slides were then cool down to room temperature, blocked with pre-antibody blocking solution for a period of time, then incubated with the HPV antibodies. The slides were then washed 3 to 5 times with PBS or H2O, or other solution to remove any unbound HPV antibody. Then the slides were incubated with the secondary antibody, for eample, anti-mouse IgG HRP, followed by appropriate substrate for detection. As an example, DAB is oxidized in the presence of peroxidase and hydrogen peroxide resulting in the deposition of a brown, alcohol-insoluble precipitate at the site of enzymatic activity. The precipitate may range in color from a light golden brown to dark golden brown depending upon the amount of enzyme present. The golden brown precipitate viewed under a microscope indicates the specific binding of HPV antibodies with HPV proteins present in the cells of the tissue section on the slide. The assay can be performed at room temperature or higher temperature to accelerate the binding reaction. This HPV IHC assay can be performed manually, or operated by IHC automation, thus provides a powerful tool to detect HPV infection and HPV oncoproteins in situ. Therefore, the HPV IHC staining assay is very useful as a confirmatory test. For the dysplasia cells identified, HPV IHC staining provides additional information for status of HPV infection and/or expression of HPV oncoproteins. Thus, overexpression of HPV E6 and E7 oncoproteins in various stage of cervical dysplasia may indicate progression of CIN and/or cervical cancer development.

Sample selection and preparation: In order to analyze if the anti-HPV antibody provided in this invention is able to detect HPV proteins in situ from different stage of CIN or cancers, the cervical tissues to be tested on the IHC assay include HSIL consisting of CIN2 (stage 2 of Cervical Intraepithelial Neoplasia with lesions appearing moderate) and CIN3 (stage 3 of Cervical Intraepithelial Neoplasia with lesions appearing severe), and invasive cancer consisting of squamous cell carcinoma (SCC, the most common carcinoma in cervical cancer) and adenocarcinoma (ADC, the gland type of carcinoma). Paraffin tissues blocks sectioned into 4 microns were placed on slide and baked at 60C overnight. Deparaffin/hydrate sections were unmasked followed by standard IHC staining procedures. Purified monoclonal antibody against HPV proteins were diluted to use as the primary antibody. Staining procedure is followed by secondary antibody solution, washing, followed by appropriate substrate reagent to each section.. As soon as the sections develop, immerse slides in dH2O, counterstain sections with hematoxylin, dehydrate and mount coverslips.

Tissue Microarry: In order to perform homogeneous assay for many samples in one reaction, tissue microarray was generated to spot many samples on one slide. To process toal of 84 samples from CIN2, CIN3, or invasive cancers, three tissue microarrays were prepared: One contains 30 individual CIN 2 and their peripheral normal epithelia, One contains 30 individual CIN 3 and their peripheral normal epithelia, One contains12 cervical squamous cell carcinomas and their normal epithelial counterparts, and 12 adenocarcinomas and their normal epithelial counterparts, vaginal or cervical mucosa of at least 15 mm away from the gross tumor border.. One representative tissue spot for neoplasia and another one spot representing its normal counterpart were taken for each CIN case. For the case of invasive cancer, 2 spots of tumor tissue and one spot of the normal counterpart were taken. A 2 mm round tissue spot was retrieved from the corresponding paraffin-embedded tissue block after taking a tissue slide for HPV DNA typing.

HPV DNA test: HPV DNA typing of each case was identified by a modified MY11/GP6+ PCR-based reverse-blot assay using EasyChip® HPV blot or a HR-HPV chip, which contained 13 type-specific oligonucleotides on a nylon membrane. Total cellular DNA was used as source of nucleic acid for amplification followed by hybridization for detection.

IHC score and data interpretation: The staining of each dot on the tissue microarray slide was viewed by certified anatomy pathologist under a microscope. Areas of tumor cells or dysplasia cells were looked up to find the percentage of cells stained, with staining intensity of score 0-3. Adjacent normal epithelium or nomal tissue 15 mm away from its corresponding dysplasia or tumors was also scored. All data were scored by certified pathologist. Stained percentage 10% was used as cut off to determine positive or negative of the assay. All data were shown as Tables 1-17.

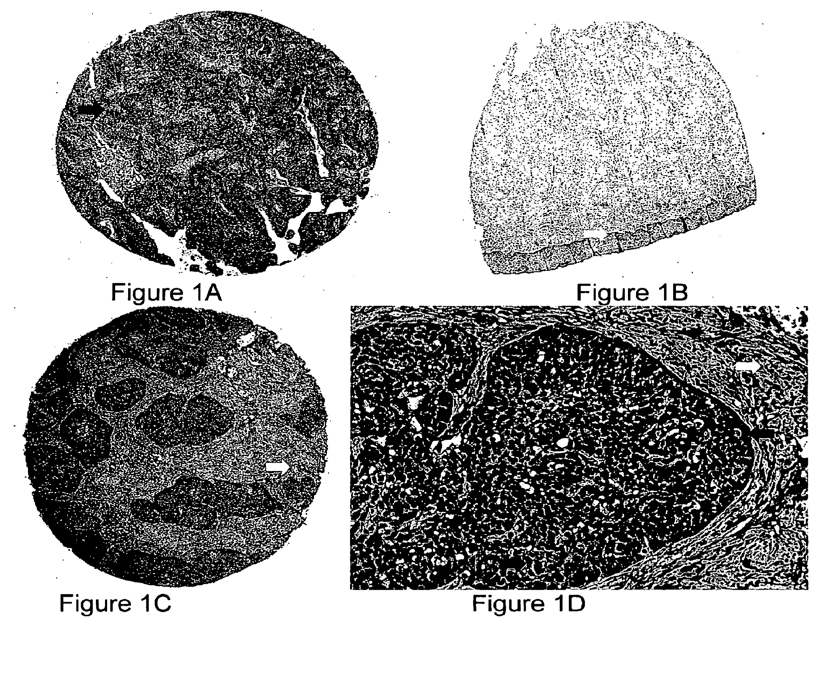

To demonstrate detection of HPV proteins in invasive cervical cancer, tissues of squamous cell carcinoma (SCC), appearing to be the most common cervical cancer, were processed to be performed on HPV IHC assay. Figure 1A-1D show IHC staining of squamous cell carcinoma tissue using a mouse monoclonal HPV E7 antibody. Figure 1A shows the representative image of the SCC tissue from tissue microarray stained by IHC using an anti-HPV E7 mouse monoclonal antibody. Figure 1B shows the representative image of the normal epithelium (15 mm away from the tumor tissue) of the SCC subject from Figure 1A. Figure 1C shows the representative image of another SCC sample from tissue microarray stained by IHC using the same anti-HPV E7 antibody. Figure 1D shows the magnified representative image of the tumor cells stained by the same anti-HPV E7 antibody in cytoplasm from Figure 1C. Results indicate expression of E7 oncoprotein can be detected in the tumor cells of SCC tissue. Solid Black arrows indicate the specific staining of E7 protein in tumor cells, while empty clear arrows indicate the normal cells with no staining. These results demonstrate that in situ prescence of HPV E7 protein from SCC cervical tissues can be detected by the mouse monoclonal anti-HPV E7 antibody used in the IHC assay.

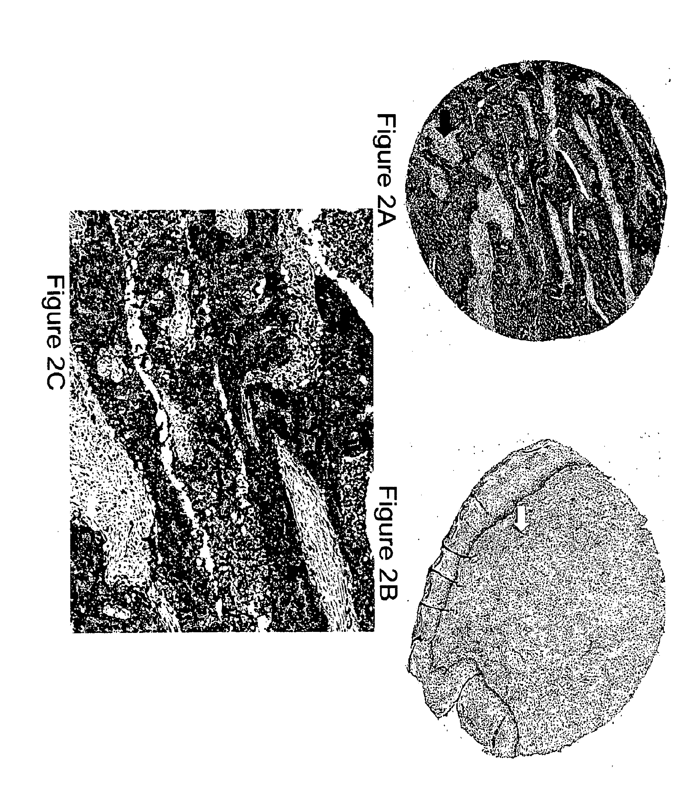

To demonstrate detection of HPV proteins in another type of invasive cervical cancer, Figures 2A-2C show IHC staining of cervical adenocarcinoma using the same mouse monoclonal HPV E7 antibody. Results indicate expression of E7 oncoprotein can be detected in the tumor cells of adenocarcinoma tissue. Solid Black arrows indicate the specific staining of E7 protein in tumor cells, while empty clear arrows indicate the normal cells with no stain. Figure 2A shows the representative image of the tumor cells of adenocarcinoma (ADC) sample stained by IHC using the same anti-HPV E7 monoclonal antibody shown in Figure 1. Figure 2B shows the representative image of the corresponding normal epithelium (15 mm away from the tumor) of the ADC sample from Figure 2A. Figure 2C shows the magnified representative image of the cytoplasm staining of adenocarcinoma tumor cells from Figure 2A. Highly magnified images indicate localization of the E7 proteins expressed in the cytoplasm of tumor cells, but not in the normal epithelium, or other cells including stroma cells. These data demonstrate the IHC staining by the E7 monoclonal antibody can detect tumor cells of adenocarcinoma showing similar staining pattern found in SCC.

To analyze the HPV IHC results from each subject of invasive cancer, Tabel 1 shows data from 24 cases of invasive cancer samples with IHC score for staining of cytoplasm (C), and nucleus (N) using C, or N followed by the % of staining using the anti-HPV E7 antibody shown in Figure 1A-1D& Figure 2A-2C. Additional anti-HPV antibodies including another anti-E7 antibody, Anti-HPV E6 antibody like MAb1 and MAb 7 and anit-HPV L1 antibody were also also tested on the same tissue microarray. To demonstrate the IHC staining by various anti-HPV antibodies, IHC score from cytoplasm staining of tumor cells using other anti-HPV antibodies was also shown. Results of HPV DNA typing were also shown in the table for its corresponding case.

As data shown in Table 1, both nucleus and cytoplasmic staining are found in all the subjects of tumor cells from SCC and ADE stained by the anti-E7 antibody. However, there is more staning (percentage stained) found in the cytoplasm of tumor cells comparing the staining of nuclear of tumor cells. The detection of HPV E7 protein in its adjacent normal epithelium cells was only found in nucleus, but not found in the cytoplasm of the epithelial cells. The staining of cytoplasm appears most distiguishable in tumor cells compared to its corresponding normal adjacent cells. These data demonstrate expression of HPV E7 proteins was detected in the cytoplasm and nuclear of tumor cells of SCC and ADE tissues. The localization of the E7 proteins expressed in the cytoplasm of tumor cells, but not in the normal epithelium or stroma cells appears tumor specific. HPV E7 proteins present in the nucleus of normal adjacent epithelium and tumor cells detected by the anti-HPV E7 antibody indicate HPV infection with oncoproteins expression. Similar staining pattern was also found when used other anti-HPV antibodies as shown in Table 1. Data indicate that the HPV IHC assay as described herein can detect HPV early gene such as E6, E7, and late gene such as L1 proteins present in the tumor cells of cervical cancer tissues.

Comparing the results of HPV IHC to the HPV DNA typing, the anti-E7 antibody reacts positively with all the HPV types present in the samples tested. For example, the anti-E7 monoclonal antibody as described herein can detect single HPV infection by at least HPV-16, HPV-18, HPV-33, HPV-45, etc, which are cancer-related HPV types (high risk HPV types). The single anti-E7 monoclonal antibody can also detect HPV infection by two or more HPV types, such as the combination of HPV 11, HPV-16, HPV-18, HPV-52, HPV-58, HPV-51, HPV-59, etc., which include high risk, low risk, and non-oncogenic α-papillomaviruses. However, infection by multiple HPV types contains at least one type is high-risk HPV type. These data indicate that the anti-E7 antibody described in this invention is non-type specific, thus provides a powerful tool to detect HPV E7 proteins from most common high-rsik HPV types in the cervical cancer.

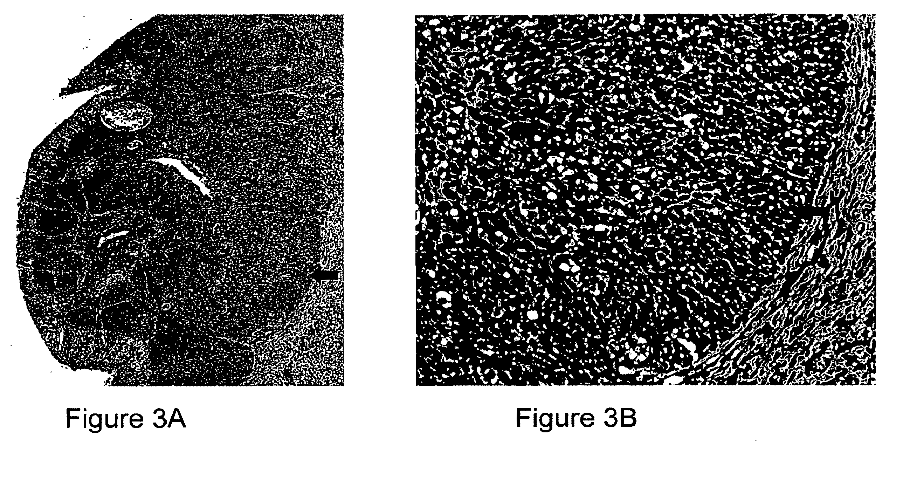

To demonstrate detection of HPV proteins in non-invasive with severe dysplasia cervical tissue (HSIL; high grade squamous intraneoplasm lesion, stage 3), Figures 3 show IHC staining of CIN3 tissue using the same mouse monoclonal HPV E7 antibody. Results indicate expression of E7 oncoprotein can be detected in the stage 3 of CIN tissue. Solid Black arrows indicate the specific staining of E7 protein in dysplasia cells, while empty clear arrows indicate the normal cells with no stain. Figure 3A shows the representative image of the dysplasia cells of a cervical intraepithelial neoplasm (CIN3) tissue stained by IHC using the same anti-HPV E7 antiobdy shown in Figure 1 and Figure 2 for invasive cancer tissue. Figure 3B shows the representative image of the adjacent normal epithelium of the CIN3 tissue of Figure 3A. These results demonstrate that in situ prescence of HPV E7 protein from CIN3 cervical tissues can be detected by the mouse monoclonal anti-HPV E7 antibody used in the IHC assay described herein.

**Table 1: IHC staining results (stained %) and HPV DNA typing for 12 SCC biopsy samples and 12 ADC biopsy samples (C: Cytoplasmic; N: Nucleus; Dys: dysplasia or tumor cells).**

| Sample # | HPV type | Anti-E7 | | | | Another anti-E7 | Anti-E6 | Another anti-E6 | Anti-L1 |
|---|---|---|---|---|---|---|---|---|---|
| | | Dys (%stained) | | Normal epith. (% stained) | | Dys (%) | Dys (%) | Dys. (%) | Dys. (%) |
| | | **C** | N | C | N | **C** | **C** | **C** | **C** |
| SCC-1 | 18 | **85** | 85 | 0 | 20 | **12.5** | **10** | **70** | **55** |
| SCC-2 | 16, 52 | **90** | 85 | 0 | 25 | **15** | **15** | **10** | **55** |
| SCC-3 | 16 | **60** | 65 | 0 | 40 | **5** | **0** | **10** | **20** |
| SCC-4 | 16 | **92** | 50 | 0 | 40 | **5** | **0** | **10** | **85** |
| SCC-5 | 16, 52, 58 | **92** | 55 | 0 | 50 | **20** | **5** | **15** | **88** |
| SCC-6 | 18, 52, 58 | **90** | 60 | | | **25** | **18** | **10** | **70** |
| SCC-7 | 16, 52 | **92** | 75 | 0 | 30 | **30** | **5** | **10** | **20** |
| SCC-8 | 16, 58 | **10** | 10 | 0 | 5 | **0** | **0** | **10** | **50** |
| SCC-9 | no DNA | **95** | 60 | 0 | 40 | **25** | **8** | **15** | **8** |
| SCC-10 | 18 | **92** | 65 | 0 | 60 | **45** | **25** | **20** | **65** |
| SCC-11 | 16, 58 | | | 0 | 80 | **5** | | 0 | **0** |
| SCC-12 | 33 | **95** | 90 | 0 | 0 | **30** | **1** | **20** | **55** |
| ADE-1 | 16,18 | **30** | 20 | 0 | 50 | **15** | **25** | **20** | **82** |
| ADE-2 | no DNA | **62** | 40 | 0 | 30 | **35** | **70** | **35** | **78** |
| ADE-3 | 16 | **20** | 30 | 0 | 20 | **35** | **55** | | **60** |
| ADE-4 | 16, 18 | **80** | 80 | 0 | 0 | **10** | **5** | **0** | **90** |
| ADE-5 | 51, 52 | **95** | 80 | 0 | 50 | **10** | **70** | **15** | **92** |
| ADE-6 | 11, 16, 52 | | | 0 | 40 | **5** | **0** | **0** | **15** |
| ADE-7 | 18 | **50** | 40 | 0 | 60 | **25** | **20** | **20** | **75** |
| ADE-8 | 18 | **85** | 60 | 0 | 40 | **15** | **50** | **15** | **82** |
| ADE-9 | 45 | **82** | 55 | 0 | 30 | **30** | **2** | **20** | **40** |
| ADE-10 | 18 | **15** | 10 | 0 | 40 | **15** | **15** | **5** | **70** |
| ADE-11 | 18, 59 | **70** | 0 | 0 | 50 | **15** | **8** | **5** | **65** |
| ADE-12 | 18 | | | | | | | | **30** |

Table 2 shows HPV IHC results from 30 human subjects, confirmed by pap smear as CIN3 samples: staining of cell membrane (M), cytoplasm (C), and nucleus (N) using M, C, or N followed by the % of staining with the anti-E7 antibody. Additional anti-HPV antibodies including Anti-HPV E6 antibody like MAb1 and MAb 7 and anit-HPV L1 antibody were also also tested on the same tissue microarray. To demonstrate the IHC staining by various anti-HPV antibodies, IHC score from cytoplasm staining of tumor cells using other anti-HPV antibodies was also shown. Results of HPV DNA typing were also shown on the table for its corresponding case. Nucleus staining are found in the dysplasia cells of all the CIN3 samples tested while only certain proportion of cases found staining of cytoplasm by the anti-E7 antibody. The results indicate that there is more staning found in the cytoplasm than in the nuclear of dysplasia cells. As shown previously in invasive cancer tissues, HPV E7 protein in its adjacent normal epithelium cells was only found in nucleus, but not found in the cytoplasm of the epithelial cells. The staining of cytoplasm appears most distiguishable in dysplasia cells compared to its corresponding normal adjacent cells. The localization of the E7 proteins expressed in the cytoplasm of dysplasia cells, but not in the normal epithelium or stroma cells appears HSIL specific. These data demonstrate expression of HPV E7 proteins can be detected in the cytoplasm and nuclear of dysplasia cells of CIN3 tissues.

HPV E7 proteins present in the nucleus of normal adjacent epithelium and dysplasia cells detected by the anti-HPV E7 antibody indicate HPV infection with oncoproteins expression. For the cases with high level expression of HPV E7 proteins detected in the cytoplasm of dysplasia cells, it suggests risk of dysplasia progression. Similar staining pattern was also found when used other anti-HPV antibodies. Data indicate that the HPV IHC assay as described herein can detect HPV early gene such as E6, E7, and late gene such as L1 proteins present in the dysplasia cells of CIN3.

Comparing the results of HPV IHC to the HPV DNA typing, the anti-E7 antibody reacts positively with all the HPV types present in the samples tested. For example, the anti-E7 monoclonal antibody as described herein can detect single HPV infection by at least HPV-16, HPV-18, HPV-31, HPV-33, HPV-39, HPV-58, etc, which are cancer-related HPV types (high risk HPV types). The single anti-E7 monoclonal antibody can also detect HPV infection by two or more HPV types, such as the combination of HPV-16, HPV-18, HPV-33, HPV-39, HPV-52, HPV-58, etc., which include most common high-risk HPV. Data indicate that the anti-E7 antibody is non-type specific, thus provides a powerful tool to detect HPV E7 proteins from most common high-rsik HPV types in the CIN3 tissues.

**Table 2: IHC staining results (stained % and score; 0-3) and HPV DNA typing of 30 CIN 3 samples (M: Membrane; C: Cytoplasmic; N: Nucleus; Dys: Dysplasia).**

| ID # | HPV type | anti-E7 | | | | | | Anti-E6 | Another anti-E7 | Anti-L1 |
|---|---|---|---|---|---|---|---|---|---|---|
| | | Dysplasia (%stained) | | | Normal epithelium (%stained) | | | Dys. (%) | Dys. (%) | Dys. (%) |
| | | M | **C** | N | M | **C** | N | **Cyto** | **Cyto** | **Cyto** |
| 31 | 33 | 0 | **80** | 80 | 0 | **0** | 50 | **70** | **40** | **80** |
| 32 | 16 | 0 | **80** | 80 | | | 60 | **0** | **0** | **5** |
| 33 | 16, 58 | | | | 0 | **0** | 60 | | | |
| 34 | 31 | 0 | **50** | 70 | 0 | **0** | 50 | **0** | **0** | **10** |
| 35 | 16, 39 | 0 | **70** | 90 | 0 | **0** | 40 | **0** | **10** | **30** |
| 36 | 31 | 0 | **70** | 60 | 0 | **0** | 50 | **0** | **20** | **20** |
| 37 | 39 | 0 | **0** | 40 | 0 | **0** | 0 | **0** | **0** | **0** |
| 38 | 16 | | | | 0 | **0** | 40 | | | |
| 39 | 16 | 0 | **60** | 70 | 0 | **0** | 40 | **0** | | **0** |
| 40 | 58 | 0 | **90** | 90 | 0 | **0** | 50 | **50** | **0** | **30** |
| 41 | 16 | 0 | **0** | 50 | 0 | **0** | 50 | **0** | **20** | **20** |
| 42 | 16 | 0 | **70** | 70 | 0 | **0** | 30 | **0** | **0** | |
| 43 | 33 | 0 | **0** | 90 | 0 | **0** | 50 | **0** | **0** | **5** |
| 44 | 52 | 0 | **70** | 80 | 0 | **0** | 50 | **70** | **10** | **50** |
| 45 | 51, 52 | 0 | **90** | 90 | 0 | **0** | 30 | **80** | **50** | **10** |
| 46 | 16 | 0 | **0** | 80 | 0 | **0** | 50 | **0** | **0** | **5** |
| 47 | 16 | 0 | **60** | 80 | 0 | **0** | 50 | **30** | **10** | **20** |
| 48 | 16, 58 | 0 | **0** | 80 | 0 | **0** | 50 | **0** | **0** | **10** |
| 49 | 31 | 0 | **80** | 60 | | | 50 | **70** | **40** | **40** |
| 50 | 16 | 0 | **0** | 60 | 0 | **0** | 30 | **0** | **20** | **20** |
| 51 | 6 | | | | 0 | **0** | 20 | | **0** | |
| 52 | 16, 18, 33, 39 | 0 | **0** | 20 | 0 | **0** | 30 | **0** | **0** | **0** |
| 53 | 51, 52, 58 | 0 | **70** | 60 | 0 | **0** | | **60** | **60** | **40** |
| 54 | 16,45 | 0 | **0** | 70 | 0 | **0** | 50 | **0** | **20** | **20** |
| 55 | 16 | 0 | **0** | 75 | 0 | **0** | 50 | **0** | **0** | **0** |
| 56 | 33, 52 | 0 | **0** | 80 | 0 | **0** | 50 | **0** | **0** | **10** |
| 57 | 16 | 0 | **0** | 50 | 0 | **0** | 40 | **0** | **0** | **0** |
| 58 | 33 | 0 | **0** | 80 | 0 | **0** | | **0** | **20** | **10** |
| 59 | 16 | 0 | **0** | 60 | 0 | **0** | 20 | **0** | **10** | **5** |
| 60 | 16, 52, 58 | 0 | **70** | 80 | 0 | **0** | 50 | **70** | **0** | **20** |

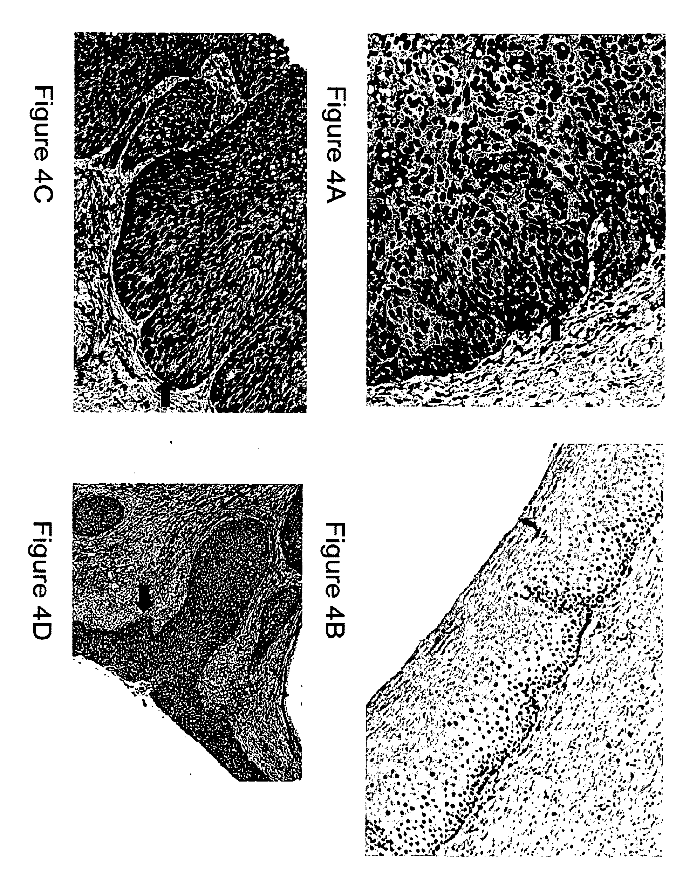

To demonstrate detection of HPV proteins in HSIL with moderate dysplasia (stage 2 of CIN), Figures 4 show IHC staining of CIN2 tissue using the mouse monoclonal anti-HPV E6 antibody. Results indicate expression of E6 oncoprotein can be detected early in the stage of CIN2. Figure 4A shows the representative image of the dysplasia cells of cervical intraneoplasm (CIN2) tissues stained by immunohistocytostaining (IHC) using the anti-E6 monolonal antibody. Figure 4B shows the representative image of the adjacent normal epithelium from the dysplasia tissue of the CIN2 sample of Figure 4A. Figure 4C shows the representative image of the dysplasia epithelium of another CIN2 sample stained by IHC using the same anti-E6 monolonal antibody. Figure 4D shows the magnified representative image of the dysplasia epithelium in Figure 4C. Solid Black arrows indicate the specific staining of E6 protein in dysplasia cells, while empty clear arrows indicate the normal cells with no stain. Similar staining pattern of CIN2 found in CIN3 indicate localization of the E6 proteins expressed in the cytoplasm of dysplasia cells, but not in the normal epithelium, or other cells including stroma cells. These results demonstrate that in situ prescence of HPV E6 protein from CIN2 cervical tissues can be detected by the mouse monoclonal anti-HPV E6 antibody used in the IHC assay described herein.

To analyze the HPV IHC results from each subject of CIN2, Table 3 shows data from 30 cases of CIN 2 samples with IHC score for staining of cell membrane (M), cytoplasm (C), and nucleus (N) using M, C, or N followed by the % of staining with the anti-E7 antibody. Additional anti-HPV antibodies including Anti-HPV E6 antibody like MAb1 and MAb 7 and anit-HPV L1 antibody were also also tested on the same tissue microarray. Table 3 demonstrates the IHC staining by various anti-HPV antibodies, results of HPV DNA typing and IHC score from cytoplasm staining of dysplasia cells using other anti-HPV antibodies were also shown. Nucleus staining are found in the dysplasia cells of all the CIN2 samples tested while only certain proportion of cases found staining of cytoplasm by the anti-E6 or anti-E7 antibody. The results indicate there is more staining of nucleus than cytoplasm of dysplasia cells found in CIN2 samples

**Table 3: IHC staining results (stained % and score; 0-3), HPV DNA typing for 30 biopsy samples (CIN2). (M: membrane; C: cytoplasmic; N: nucleus; Dys: dysplasia)**

| ID # | HPV type | Anti-E7 | | | | | | Anti-E6 | another anti-E7 | Anti-L1 |
|---|---|---|---|---|---|---|---|---|---|---|
| | | Dysplasia (% stained) | | | Normal epithelium (%stained) | | | Dys. (%) | Dys. (%) | Dys. (%) |
| | | M | **C** | N | M | **C** | N | **Cyto** | **Cyto** | **Cyto** |
| 1 | 6 | 0 | **80** | 80 | 0 | **0** | 30 | **70** | **40** | **80** |
| 2 | 31 | 0 | **0** | 90 | | | | **0** | **40** | **0** |
| 3 | 52 | 0 | **25** | 50 | 0 | **0** | 70 | **0** | **20** | **20** |
| 4 | 16 | 0 | **0** | 40 | 0 | **0** | 30 | **0** | **5** | **0** |
| 5 | 58 | 0 | **0** | 50 | 0 | **0** | 10 | **0** | **0** | **0** |
| 6 | 52 | 0 | **80** | 70 | 0 | **0** | 50 | **0** | **5** | **0** |
| 7 | 53 | 0 | **0** | 80 | 0 | **0** | 30 | **0** | **10** | **10** |
| 8 | 52 | 0 | **50** | 90 | 0 | **0** | 20 | **60** | **10** | **20** |
| 9 | 31 | 0 | **80** | 80 | 0 | **0** | 50 | **70** | **20** | **40** |
| 10 | 16 | 0 | **50** | 80 | 0 | **0** | 50 | **60** | **20** | **10** |
| 11 | no DNA | 0 | **0** | 50 | 0 | **0** | 70 | **0** | **0** | **10** |
| 12 | 33 | 0 | **60** | 60 | 0 | **0** | 50 | **0** | **10** | **30** |
| 13 | no DNA | 0 | **70** | 80 | 0 | **0** | 70 | **0** | **20** | **10** |
| 14 | 52 | 0 | **0** | 70 | 0 | **0** | 70 | **0** | **30** | **20** |
| 15 | no DNA | 0 | **0** | 70 | 0 | **0** | 50 | **0** | **20** | **5** |
| 16 | 52 | 0 | **0** | 10 | **0** | **0** | 30 | **0** | **0** | **5** |
| 17 | 52 | 0 | **0** | 60 | 0 | **0** | 80 | **0** | **0** | **5** |
| 18 | 16 | 0 | **50** | 60 | **0** | **0** | 30 | **50** | **10** | **20** |
| 19 | 16 | 0 | **50** | 70 | | | | **0** | **10** | **20** |
| 20 | 52, 44 | 0 | **50** | 80 | 0 | **0** | 40 | **0** | **30** | **30** |
| 21 | 16 | 0 | **0** | 50 | 0 | **0** | 50 | **0** | **20** | **20** |
| 22 | 16, 18, 6 | 0 | **0** | 40 | 0 | **0** | 0 | **0** | **10** | **0** |
| 23 | 16,31 | 0 | **0** | 30 | 0 | **0** | 60 | **0** | **0** | |
| 24 | 6 | 0 | **0** | 80 | 0 | **0** | 50 | **0** | **10** | **5** |
| 25 | 16 | 0 | **0** | 10 | 0 | **0** | 60 | **0** | **0** | **0** |
| 26 | 58 | 0 | **0** | 40 | 0 | **0** | 40 | **0** | **10** | **5** |
| 27 | 16, 39, 52 | | | | 0 | **0** | 70 | | **0** | |
| 28 | 6 | 0 | **0** | 50 | 0 | **0** | 70 | **0** | **10** | **5** |
| 29 | 16 | 0 | **0** | 70 | 0 | **0** | 5 | **0** | **10** | **20** |
| 30 | 66, 68, | 0 | **0** | 30 | 0 | **0** | 60 | **0** | **10** | **0** |

. In SCC, ADC, and CIN3, it is shown that HPV E7 protein is found in its adjacent normal epithelium cells only in nucleus, but not found in the cytoplasm of the epithelial cells. The staining of cytoplasm in CIN2 using anti-E6 antibody appears most distiguishable in dysplasia cells compared to its corresponding normal adjacent cells. The localization of the E6 proteins expressed in the cytoplasm of dysplasia cells, but not in the normal epithelium or stroma cells appears HSIL specific. These data demonstrate expression of HPV E6 proteins can be detected in the cytoplasm and nuclear of dysplasia cells of CIN2 tissues. For the cases with high level expression of HPV E6 proteins detected in the cytoplasm of dysplasia cells, it may suggest dysplasia progression. Early protein such as E6, E7, and late protein, such as L1 can be detected in CIN2 dysplasia cells by HPV IHC assay as described here.

The anti-E7 antibody reacts positively with all the HPV types present in the samples tested, when the results of HPV IHC and HPV DNA typing are compared,. For example, the anti-E7 monoclonal antibody as described herein can detect single HPV infection by at least, HPV-16, HPV-18, HPV-31, HPV-52, HPV-58, etc, which are cancer-related HPV types (high risk HPV types) and HPV6, HPV 53 which are not high-risk HPV types. A single anti-E7 monoclonal antibody can also be used to detect HPV infection by two or more HPV types, including HPV6, HPV-16, HPV-18, HPV-31, HPV-39, HPV-44, HPV-52, HPV-58, HPV-66, HPV-68, etc., which include most common high-risk HPV as well as low risk HPV types. These data indicate that the anti-E7 antibody described in this invention is non-type specific, is able to detect HPV E7 proteins from common high-rsik HPV types as well as low risk types in the CIN2 tissues. It is possible that formation of dysplasia cells resulted from the expression of oncoproteins, rather than variation of different HPV types. The results may expalin regression occurred for infection by high-risk types with no detectable oncoproteins in cytoplasmic portions. Thus, the HPV IHC assay described herein provides clinical information, for detection of HPV infection and indication of dysplasia progression.

Since the staining of cytoplasm is only found in dysplasia cells distinguishable to its corresponding normal cells, cytoplasmic staining was used to demonstrate the assay performance in dysplasia progression. To compare the specific cytoplasm staining of dysplasia cells in different stages of CIN or cancer tissues, data from Table 1-3 were further analyzed to obtain the assay performance. Percentage of staining 10% or above from each subject is considered positive, otherwise is negative for the sample less than 10% stained.

**Table 4: Summary of the IHC staining results using anti-E7 on various biopsy samples during cervical cancer development and tissue lesions.**

| | Anti-E7 | dysplasia or Tumor Cyto. stain | Normal Cytoplasmic stain | IHC positive rate | Specificity | | |
|---|---|---|---|---|---|---|---|
| CIN2 | Positive | 11 | 0 | 38% | 100% | **100%** | **PPV** |
| | Negative | 18 | 28 | | | **61%** | **NPV** |
| CIN3 | Positive | 14 | 0 | 52% | 100% | **100%** | **PPV** |
| | Negative | 13 | 28 | | | **68%** | **NPV** |
| SCC | Positive | 11 | 0 | 100% | 100% | **100%** | **PPV** |
| | Negative | 0 | 11 | | | **100%** | **NPV** |
| ADC | positive | 10 | 0 | 100% | 100% | **100%** | **PPV** |
| | negative | 0 | 10 | | | **100%** | **NPV** |

**Table 5: Summary of the IHC staining results using another anti-E7 on various biopsy samples during cervical cancer development and tissue lesions.**

| | Another anti-E7 | dysplasia or Tumor Cyto. stain | Normal Cytoplasmic stain | IHC positive rate | Specificity | | |
|---|---|---|---|---|---|---|---|
| CIN2 | Positive | 21 | 16 | 72% | | **57%** | **PPV** |
| | negative | 8 | 9 | | 36% | **53%** | **NPV** |
| CIN3 | Positive | 13 | 4 | 48% | | **76%** | **PPV** |
| | negative | 14 | 25 | | 86% | **64%** | **NPV** |
| SCC | Positive | 8 | 5 | 67% | | **62%** | **PPV** |
| | negative | 4 | 6 | | 55% | **60%** | **NPV** |
| ADC | Positive | 10 | 6 | 83% | | **63%** | **PPV** |
| | negative | 2 | 6 | | 50% | **75%** | **NPV** |

As data shown in table 4, the positive rate of the assay increases with the severity of CIN shown 38%, 52% to 100% for CIN2, CIN3, and SCC or ADE respectively. Positive predictive value (PPV) and negative predictive value (NPV) were also shown in this table to demonstrate the assay specificity using percentage staining of cytoplasm. To show the expression of HPV E7 oncoproteins can be detected by another anti-E7 monoclonal antibodies, same tissue microarray wese tested and shown in Table 5 representing the assay performance of another E7 antibody with positive rate of 72%, 48%, 67%, 83% for CIN2, CIN3, SCC and Adenocarcinoma respectively. Positive predictive value (PPV) and negative predictive value (NPV) indicates the assay specificity using this antibody is not as good.

**Table 6 Summary of the IHC staining results using anti-E6 on various biopsy samples during cervical cancer development and tissue lesions.**

| | Anti-E6 | dysplasia or Tumor Cyto. stain | Normal Cytoplasmic stain | IHC positive rate | Specificity | | |
|---|---|---|---|---|---|---|---|
| CIN2 | Positive | 5 | 1 | 17% | | **83%** | **PPV** |
| | Negative | 25 | 29 | | 97% | **54%** | **NPV** |
| CIN3 | Positive | 17 | 7 | 57% | | **71%** | **PPV** |
| | Negative | 13 | 23 | | 77% | **64%** | **NPV** |
| SCC | Positive | 7 | 1 | 64% | | **88%** | **PPV** |
| | Negative | 4 | 10 | | 91% | **71%** | **NPV** |
| ADC | Positive | 9 | 0 | 75% | | **100%** | **PPV** |
| | Negative | 3 | 12 | | 100% | **80%** | **NPV** |
| total | Positive | 38 | 9 | 46% | 0 | **81%** | **PPV** |
| | Negative | 45 | 74 | 0 | 89% | 62% | NPV |

**Table 7: Summary of the IHC staining results using another anti-E6 on various biopsy samples during cervical cancer development and tissue lesions.**

| | Another anti-E6 | dysplasia or Tumor Cyto. stain | Normal Cytoplasmic stain | IHC positive rate | Specificity | | |
|---|---|---|---|---|---|---|---|
| CIN2 | positive | 5 | 0 | 17% | | **100%** | **PPV** |
| | negative | 24 | 28 | | 100% | **54%** | **NPV** |
| CIN3 | positive | 8 | 0 | 30% | | **100%** | **PPV** |
| | negative | 19 | 29 | | 100% | **60%** | **NPV** |
| SCC | positive | 11 | 0 | 92% | | **100%** | **PPV** |
| | negative | 1 | 11 | | 100% | **92%** | **NPV** |
| ADC | positive | 9 | 0 | 75% | | **100%** | **PPV** |
| | negative | 3 | 12 | | 100% | **80%** | **NPV** |
| total | positive | 33 | 0 | 41% | 0 | **100%** | **PPV** |
| | negative | 47 | 80 | 0 | 100% | **63%** | **NPV** |

To validate the expression of HPV E6 oncoproteins can be detected by anti-E6 monoclonal antibodies, same tissue microarray wese tested and shown in Table 6-7 representing the assay performance of two anti-E6 antibodies. As data shown, E6 proteins expressed in the cytoplasm of dysplasia cells from CIN2, CIN3, as well as tumor cells from invasive cancer samples can be detected by the anti-E6 antibody described in this invention. The same trend shown positive rate of the anti-E7 IHC assay increased with the severity of CIN is also found in the assay using anti-E6 antibody. The two anti-E6 antibodies show the same trend of increasing positive rate of assay over severity of CIN although one anti-E6 may give better assay performance than the other one. It is possible that MAb1 recognize different epitope from MAb7 does, thus give different assay performance. However, both monoclonal antibodies give high positive predictive value (PPV) and high negative predictive value (NPV) as shown in the tables. The overall positive rate of IHC assay using anti-E7 antibody is higher than using anti-E6 antibody. It is possible that E7 proteins are expressed earlier to serve as a biomarker for early detection of cervical cancer.

To detect the expression of L1 viral proteins present in different stage of CIN, the same tissues microarrays were also tested on IHC assay using anti-L1 antibody. IHC score from L1 staining of cytoplasm was also obtained to analyze its positivity rate on all the samples. To study the correlation of HPV early and late proteins expressed in situ in different stage of CIN, Table 8 shows the positive rate of IHC assay with E7 expression and L1 expression in the cytoplasm for CIN2, CIN3, and invasive cancers. Since E7 seems a good biomarker for early detection of cervical caner, we compare the correlation of E7 and L1 expression in various stages of CIN and invasive cancer tissues using the HPV IHC assay described in this invention. For L1 IHC positive of CIN samples, as data shown in Table 8, about 60% (9 out of 15) of CIN2, or 58% (11 out of 19) of CIN3 show positive on E7 IHC assay. For L1 cytoplasmic positive of invasive cancer samples, 100% of both SCC (11 out of 11) and adenocarcinoma (10 out of 10) are E7 cytoplasmic positive, indicating 100% correlation of E7 expression with cancer progression. Data indicate that both L1 and E7 cytoplasmic positive of CIN2/3 may have higher risk in further dysplasia progression compared to those L1 postive but E7 negative on IHC assay.

Table 8 also shows the correlation of sample positivity with E7 expression and E6 expression in the cytoplasm. As data shown, for E7 cytoplasmic positive samples, about 45% (5 out of 11) of CIN2, or 57% (8 out of 14) of CIN3 show positive on E6 cytoplasmic expression, while 100% (11 out of 11) of SCC or 90% (9 out of 10) of ADE show E6 cytoplasmic expression. These data indicate that E6 may be expressed behind E7 during early dysplasia, but co-expressed in the late stage of cervical cancer.

**Table 8: Summary of the IHC staining results using Anti-E7 compared with Anti-L1 and Anti-E6 on various biopsy samples during cervical cancer development and tissue lesions.**

| | CIN2 | | CIN3 | | SCC | | ADC | |
|---|---|---|---|---|---|---|---|---|
| Total No. of samples | L1 (+) | L1 (-) | L1 (+) | L1 (-) | L1 (+) | L1 (-) | L1 (+) | L1 (-) |
| E7 positive | **9** | 2 | **11** | 3 | **11** | 0 | **10** | 0 |
| E7 negative | 6 | **8** | 8 | **5** | 0 | **0** | 0 | **0** |
| No. of samples | E6 (+) | E6 (-) | E6 (+) | E6 (-) | E6 (+) | E6 (-) | E6 (+) | E6 (-) |
| E7 positive | **5** | 6 | **8** | 6 | **11** | 0 | **9** | 1 |
| E7 negative | 0 | **17** | 0 | **13** | 0 | **0** | 0 | **0** |

In order to have homogeneous assay conditions for all stages of samples in one reaction, additional tissue microarray was generated to spot many samples from CIN2, CIN3, and invasive cancers on the same slide. Two tissue microarrays were prepared: One contains 10 individuals each for CIN2, CIN3, and SCC and their peripheral normal epithelia. One contains 10 individuals each for CIN2, CIN3, and ADE and their peripheral normal epithelia. To confirm the HPV IHC assay is specific to HPV related dysplasia or cervical cancer, additional tissue microarray containing more than 90 samples from various normal human tissues was also tested to use as the negative control of the HPV IHC assay. The same HPV IHC assays using anti-E6 and anti-E7 antibody were applied on these tissue microarray to obtain IHC staining percentage as described above. IHC staining of 10% or above shown in nucleus and/or cytoplasm was scored as positive of the assay.

**Table 9: IHC staining results using a monoclonal anti-HPVE7 antibody on various biopsy samples during cervical cancer development and tissue lesions.**

| IHC | CIN2 | CIN3 | SCC | ADC | total |
|---|---|---|---|---|---|
| Anti-HPV E7 positive | 34 | 43 | 22 | 23 | 122 |
| Anti -HPV E7 negative | 13 | 5 | 0 | 0 | 18 |
| Positive rate | 72% | 90% | 100% | 100% | 87% |

**Table 10: Summary of the immunohistochemistry staining results using a mouse monoclonal anti-HPVE7 antibody on CIN2+ lesions compared to CIN negative samples.**

| | CIN2+ | CIN negative | |
|---|---|---|---|
| HPVE7 positive | 122 | 7 | 95% PPV |
| HPVE7 negative | 18 | 85 | 83% NPV |
| Sensitivity | 87% | | |
| Specificity | | 92% | |

All together, the IHC data from all the tissue microarrays tested herein were shown in Table 9-14. Table 9 shows IHC staining results using a mouse anti-HPVE7 antibody on various biopsy samples during cervical cancer development and tissue lesions. Data in Table 9 indicate that the presence of HPV E7 proteins in situ can be detected from various stages of cervical tissues with increasing positivity rate of assay from, CIN2, CIN3 to invasive cancer tissue like squamous cell carcinoma (SCC) or adenocarcinoma (AD). There is about 72%, and 90% positive rate for samples with CIN2 and CIN3 respectively. For cancer tissues (SCC and AD), 100% of samples stains positively by IHC using anti-HPV E7 antibody, indicating 100% of cancers expressing HPV oncogenic proteins. These data indicate the IHC assay using the HPV E7 antibody described in this invention provides a powerful tool to confirm the diagnosis of cervical cancer from the tissues in various grade.

To obtain assay performance, data from table 9 were further analyzed. Table 10 shows summary of the IHC staining results from Table 9 using normal human tissues as CIN2 negative samples. Data indicate that the IHC staining method using the anti-HPV E7 antibody provides IHC assay sensitivity of 87% for CIN2+ with specificity of 92%. These data suggest this assay can be useful to detect HPV proteins for confirming of cervical lesion CIN2 or above.

To further analyze the data, Table 11 shows summary of the immunohistochemistry staining results from Table 9 and Table 10 indicating that using the anti-HPV E7 antibody described in this invention provides immunohistochemistry assay for CIN3+ (including CIN3, and invasive cancer) with 95% sensitivity, 92% specificity and 93% of positive predictive value, and 95% of negative predictive value. These data suggest this assay can be useful for clinical application to detect HPV proteins confirming cervical lesion in different stages.

**Table 11: Summary of the immunohistochemistry staining results using a mouse monoclonal anti-HPVE7 antibody on CIN3+ lesions compared to CIN negative samples.**

| | CIN3+ | CIN negative | |
|---|---|---|---|
| HPVE7 positive | 88 | 7 | 93% PPV |
| HPVE7 negative | 5 | 85 | 94% NPV |
| sensitivity | 95% | | |
| Specificity | | 92% | |

To confirm if expression of E6 protein can be detected by immunohistochemistry assay using the anti-E6 antibody described in this invention, the same tissues microarrays were performed on IHC assay using an anti-E6 antibody. As an example of another HPV immunohistochemistry assay, Table 12-14 show results of immunohistochemistry staining using anti-HPV E6 antibody. As data shown, HPV anti-E6 gives comparable immunohistochemistry results as HPV anti-E7.

Table 12 shows immunohistochemistry staining results using a mouse anti-HPVE6 antibody on various biopsy samples during cervical cancer development and tissue lesions. Data indicate that HPV E6 protein can be detected by the mouse monoclonal anti-HPV E6 antibody used in the immunohistochemistry assay. The presence of HPV E6 proteins in situ can be detected from various stages of cervical tissues. As data shown, HPV E6 proteins are present in the samples with increasing positivity rate from, CIN2, CIN3 to cancer tissue like squamous cell carcinoma (SCC) or adenocarcinoma (AD). There is about 65%, and 70% positive rate for samples with CIN2 and CIN3 respectively. For cancer tissues, 96% of SCC samples stain positively by immunohistochemistry using anti-HPV E6 antibody, while only 71% of AD samples stain positively, indicating HPV E6 oncoproteins expressing more predominately in SCC than in AD. These data indicate the immunohistochemistry assay using the HPV E6 antibody described in this invention provides a tool to confirm the diagnosis of cervical cancer from the tissues in various grade.

**Table 12: Immunohistochemistry staining results using a monoclonal anti-HPVE6 antibody on various biopsy samples during cervical cancer development and tissue lesions.**

| IHC | CIN2 | CIN3 | SCC | AD | total |
|---|---|---|---|---|---|
| HPVE6 positive | 32 | 35 | 23 | 17 | 107 |
| HPVE6 negative | 17 | 15 | 1 | 7 | 40 |
| sensitivity | 65% | 70% | 96% | 71% | 73% |

**Table 13: Summary of the immunohistochemistry staining results using a mouse monoclonal anti-HPVE6 antibody on CIN2+ lesions compared to CIN negative samples.**

| | CIN2+ | CIN negative | |
|---|---|---|---|
| HPVE6 positive | 107 | 12 | 90% PPV |
| HPVE6 negative | 40 | 80 | 67% NPV |
| Sensitivity | 73% | | |
| Specificity | | 87% | |

To further analyze the data, Table 13 shows summary of the IHC staining results from Table 11 and Table 12. As data indicated, using the anti-HPV E6 antibody provides immunohistochemistry assay sensitivity of 73% for CIN2+ with specificity of 87%. These data suggest this assay can be useful for clinical application to detect HPV proteins confirming cervical lesion in different stages.

**Table 14: Summary of the immunohistochemistry staining results using a mouse monoclonal anti-HPVE7 antibody on CIN3+ lesions compared to CIN negative samples.**

| | CIN3+ | CIN negative | | |
|---|---|---|---|---|
| HPVE6 positive | 75 | 7 | 91% PPV | |
| HPVE6 negative | 23 | 85 | 79% NPV | |
| sensitivity | 77% | | | |
| Specificity | | 92% | | |

To further analyze the data, Table 14 shows summary of the immunohistochemistry (IHC) staining results from Table 13 indicating the IHC method using the anti-HPV E6 antibody described in this invention provides immunohistochemistry assay for CIN3 or above with sensitivity of 77% specificity of 92% and 91% PPV, and 79% NPV. Data suggest this assay is useful for clinical application to detect HPV proteins confirming cervical lesion in different stages.

### 5. The HPV ICC assays.

Sample preparation: Two types of samples including standard conventional pap smear sample and liquid based cytology samples can be used for the ICC assay. The cervical scrape cells collected from direct smear on slides or liquid based solution were divided into two parts, one for cytological papanicolaou staining, the other one for immunocytochemical staining using HPV antibodies described in this invention. For Pap smear results from papanicolaou staining, samples were scored from 0-17 as one (1) to three (3) are considered as normal and four (4) and above as abnormal. The abnormal cells include different stage of squamous cells in development of dysplasia or lesions. For examples, LSIL: Low grade of Squamous Intraepithelial Lesion, HSIL: High grade of Squamous Intraepithelial Lesion, CIN 1: Cervical Intraepithelial Neoplasia; mild cell abnormalities, CIN2: Cervical Intraepithelial Neoplasia with lesions appearing more aggressive, CIN3: Cervical Intraepithelial Neoplasia with aggressive form of dysplasia. Invasive cancers may include squamous cell carcinoma (SCC) adenocarcinoma (ADC), and others. For abnormal cells are underdetermined, ASCUS: Atypical Squamous Cells of Undetermined Significance; unusual or atypical cells in pap smear, may be inconsequential and significance is underdetermined. AGUS: Atypical Glands of Undetermined Significance. For the abnormal cells identified, HPV ICC staining may provide additional information for status of HPV infection and/or expression of HPV oncoproteins. Therefore, HPV ICC staing assay is very useful to detect LSIL or HSIL abnormal cells, and/or for those underdetermined abnormal cells like ASCUS, or AGUS, comparing to the papanicolaou staining.

As an example of immunoassay using ICC methods, cells from cervical scrapes were directly smeared on the slides, or collected into liquid based solution, centrifuged, washed, and immunostained followed by the ICC procedure described herein. Cervical scrapes collected by Liquid based solution were processed according to the manufacture instruction. The cervical cells were then processed by cytospin or thin prep techniques into monolayer. The thin layer of cells on slide were then fixed and stained by anti-HPV antibodies followed by HPV ICC protocol. Stained cells are visualized under microscope.

In one embodiment, a kit for performing an ICC assay is provided. The kit may include an pre-antibody blocking solution, post-antibody blocking solution, an anti-HPV antibody as the primary antibody, an anti-mouse or anti-rabbit immunoglobulins conjugated with HRP or biotin, or other agents as secondary antibody, a solution containing appropriate agents used as substrate for the secondary antibody to be detected.

The anti-HPV antibodies may also be directly tagged with HRP or biotin, or other agents to be detected following appropriate agents used as substrate. The pre-antibody blocking solution may contain certain proteins or BSA, or serum or other agents to block the cells from nonspecific binding of antibody. The post blocking solution may contain similar solution as the pre-antibody blocking solution with less proteins or serum to be used along with primary antibody incubation. The solution containg HPV antibodies may be in concentrated form, or may be in diluted form as ready to use reagent. The solution containing secondary antibodies may be in concentrated form, or may be in diluted form as ready to use reagent. The solution containing appropriate agents used as substrate may include DAB (3.3'-diaminobenzidine) as one component, or two components, or AEC (3-Amino-9-Ethylcarbazole) substrate as one component, or two components, or other substrates.

Once the human cells from cervical scrapes are processed and fixed into a monolayer or thin layer of cells on the slide, the Immunocytochemistry (ICC) assay is performed by blocking the slides with pre-antibody blocking solution for a period of time prior to incubate with the HPV antibodies. The slides were then washed 3 to 5 times with PBS or H2O, or other solution to get rid of any unbound HPV antibody. Then the slides were incubated with the secondary antibody, for eample, anti-mouse IgG HRP, followed by appropriate substrate for detection. As an example, DAB is oxidized in the presence of peroxidase and hydrogen peroxide resulting in the deposition of a brown, alcohol-insoluble precipitate at the site of enzymatic activity. The precipitate may range in color from a light golden brown to dark golden brown depending upon the amount of enzyme present. The golden brown precipitate viewed under a microscope indicates the specific binding of HPV antibodies with HPV proteins present in the cells. The assay can be performed at room temperature or higher temperature to accelerate the binding reaction. This HPV ICC assay can be performed manually, or operated by ICC automation, thus provides a powerful tool to screen for HPV infection and detection of HPV oncoproteins in situ localized in the epithelium cells from cervical scrapes.

To demonstrate the HPV ICC assay can be applied to different stage of dysplasia cells, samples from early, intermediate, or late stage of neoplasia are all tested. These samples include but not limited to, for example, early stage like LSIL, or CIN1, or ASCUS, or intermediate stage like CIN2, CIN3, or HSIL, or late sage like SCC or ADE or others. To demonstrate the ICC assay described herein can be used to stain for various sample source, or from various stage in various liquid based solution, different stage of samples in different liquid based solution were also prepared to perform ICC assay in this invention.

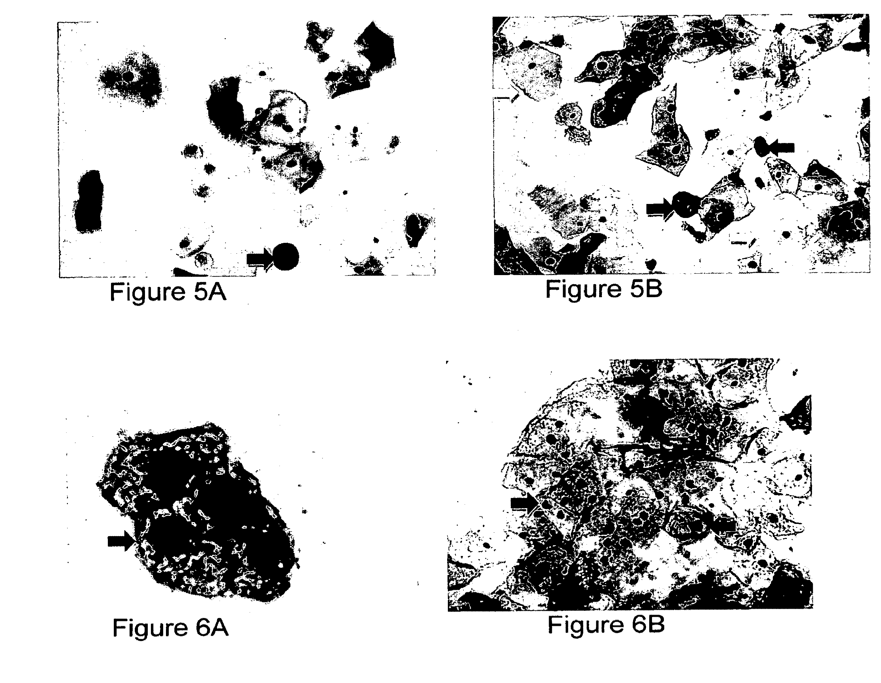

To demonstrate HPV ICC assays are useful to identify abnormal cells underdetermined by standard cytological papanicolaou staining, for example, ASCUS (Atypical Squamous Cells of Undetermined Significance; unusual or atypical cells in pap smear, may be inconsequential and significance is underdetermined) or AGUS (Atypical Glands of Undetermined Significance), the HPV ICC assays are performed to test ASCUS samples. As shown in Figure 5A, the results of ICC assay demonstrate that certain cervical scrape cells diagnosed as ASCUS by papanicolaou staining can be ICC stained positively using an anti-E6 monoclonal antibody. Figure 5B shows the results of ICC assay from the same sample shown in Figure 5A to demonstrate certain cervical scrape cells (diagnosed as ASCUS by papanicolaou staining) can be ICC stained positively using an anti-E7 monoclonal antibody. As shown in Figure 5A and Figure 5B, the abnormal cell with high N/C (nuclear/cytoplasm) ratio (indicated as black arrow) was stained positively while the normal cells (big, irregular cell shape with small nuclear) stain negatively as indicated by the white arrow. Both Figure 5A and Figure 5B demonstrate HPV E6 and HPV E7 proteins can be detected in the abnormal cells from sample with pap smear ASCUS. These results indicate that this ASCUS sample containing HPV infected cells with E6 and E7 oncogenic proteins expressed, thus can be detected in situ using the mouse monoclonal anti-HPV E6 and the mouse anti-HPV E7 monoclonal antibody respectively, by the ICC assay described in this invention.

To demonstrate the HPV ICC assay can detect HSIL cells, Figure 6A shows cervical scrape cells diagnosed as CIN2 by papanicolaou staining prepared in another liquid base solutions can be ICC stained positively using an anti-E7 monoclonal antibody. As shown in Figure 6A, the CIN2, HSIL abnormal cells in the form of connecting each other with high N/C (nuclear/cytoplasm) ratio (indicated as black arrow) was stained positively to the nucleus and cytoplasm. These results demonstrate that HPV E7 protein present in situ can be detected in the abnormal cells from intermediate stage of neoplasm, in various source of liquid based solution using the mouse monoclonal anti-HPV E7 described here in the ICC assay.

As an another example, Figure 6B shows another CIN2 sample of cervical scrape cells prepared in another liquid base solutions can be ICC stained positively using an anti-E6 monoclonal antibody. As shown in Figure 6B, the CIN2, HSIL abnormal cells in the form of connecting each other with high N/C (nuclear/cytoplasm) ratio (indicated as black arrow) was stained positively to the nucleus and cytoplasm. These results demonstrate that HPV E6 protein present in situ can be detected in the abnormal cells from intermediate stage of neoplasm, in various source of liquid based solution using the mouse monoclonal anti-HPV E6 described here in the ICC assay.

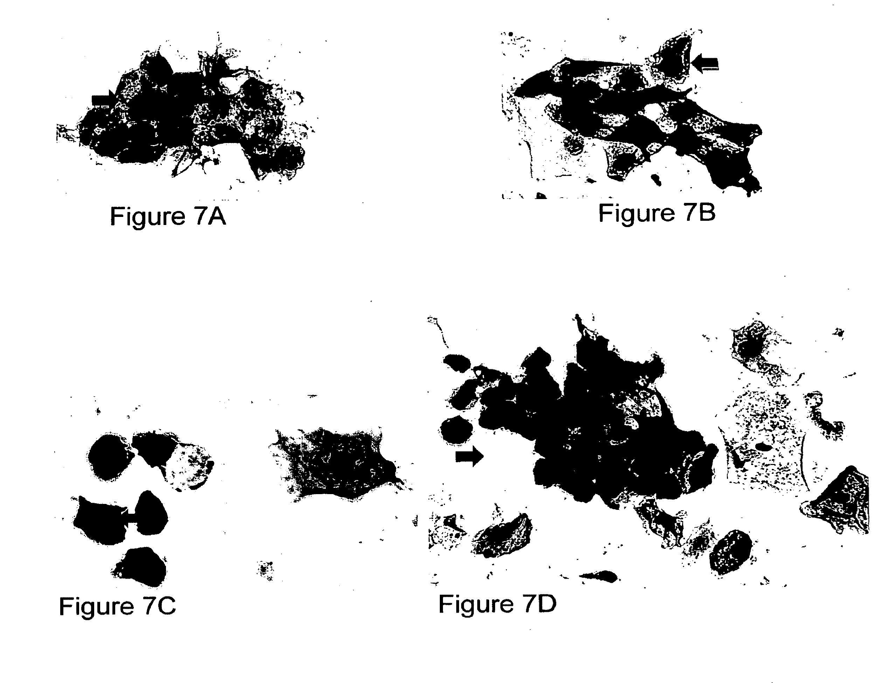

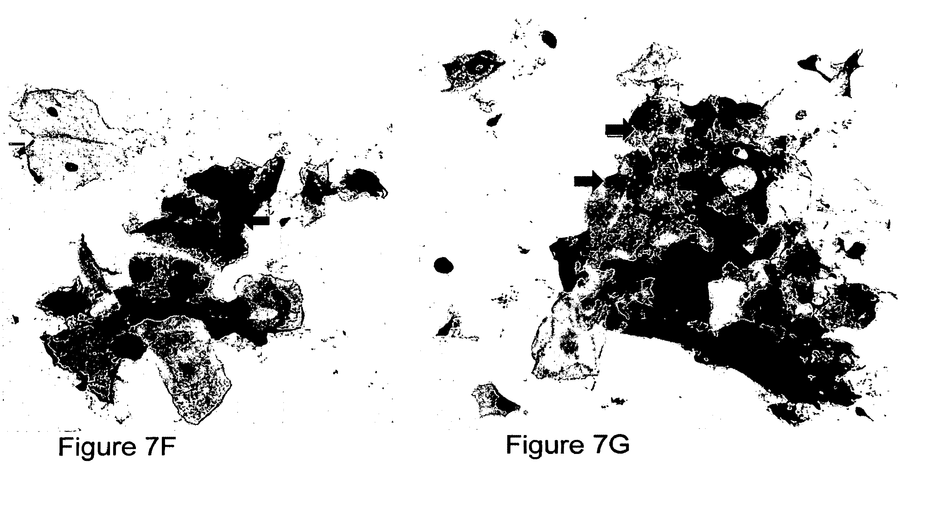

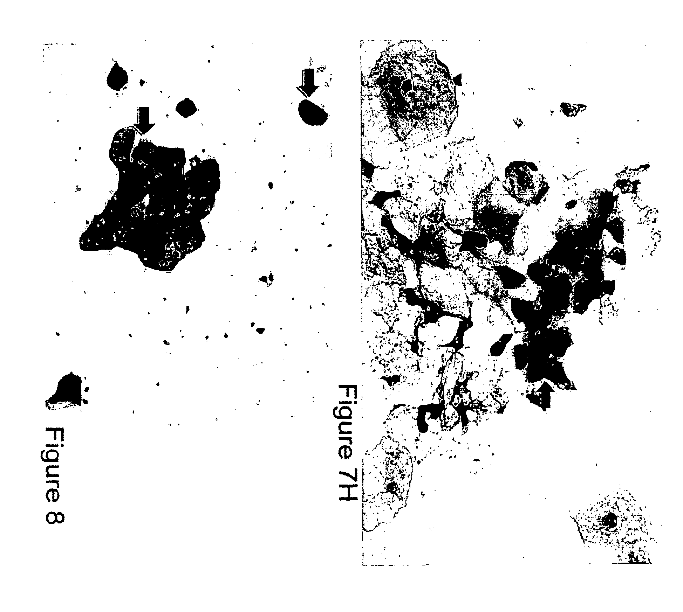

To demonstrate the ICC assay described herein can identify intermediate to late stages of cervical intraneoplasm (CIN) cells in liquid based solution, CIN3 cervical scrape samples in different liquid based solution were also prepared to perform ICC assay described in this invention. Figure 7A shows certain cervical scrape cells (diagnosed as CIN3 by papanicolaou staining) can be ICC stained positively using an anti-E6 monoclonal antibody. Figure 7B-7E shows another CIN3 using the same anti-HPV E6 mouse monoclonal antibody. As Figures shown, the CIN3, HSIL abnormal cells in the form of connecting each other with high N/C (nuclear/cytoplasm) ratio (indicated as black arrow) was stained positively to the nucleus and cytoplasm. These results demonstrate that HPV E6 protein present in situ can be detected in the abnormal cells from intermediate/late stage of neoplasm, in various source of liquid based solution using the mouse monoclonal anti-HPV E6 described here in the ICC assay..These results demonstrate that HPV E6 protein present in situ can be detected in the abnormal cells from CIN3 of liquid based solution using the mouse monoclonal anti-HPV E6 described here in the ICC assay.

Figure 7E shows the results of of ICC staining from the same CIN3 sample shown in Figre 7B using an anti-HPV E7 mouse monoclonal antibody. Figure 3F shows another image of the same ICC staining results shown in Figure 3E using the same anti-HPV E7 mouse monoclonal antibody. As Figures shown, the CIN3, HSIL abnormal cells in the form of connecting each other with high N/C (nuclear/cytoplasm) ratio (indicated as black arrow) was stained positively to the nucleus and cytoplasm. These results demonstrate that HPV E7 protein present in situ can be detected in the abnormal cells from intermediate stage of neoplasm, in various source of liquid based solution using the mouse monoclonal anti-HPV E7 described here in the ICC assay. These results demonstrate that HPV E7 protein present in situ can be detected in the abnormal cells of CIN3 from liquid based solution using the mouse monoclonal anti-HPV E7 described here in the ICC assay. To confirm p16 is also overexpressed in the late stage of neoplasm, the same CIN3 samples was used to do ICC staining using an anti-p16 mouse monoclonal antibody. Figure 7G shows the results of ICC staining from the same CIN3 sample shown in Figre 7B-7F using an anti-p16 mouse monoclonal antibody. As Figures shown, the CIN3, HSIL abnormal cells in the form of connecting each other with high N/C (nuclear/cytoplasm) ratio (indicated as black arrow) was stained positively to the nucleus and cytoplasm. These results demonstrate that p16 protein present in situ can be detected in the abnormal cells from intermediate to late stage of neoplasm. These results demonstrate that HPV E6, HPV E7, and p16 protein present in situ can be detected in the abnormal cells from CIN3 of liquid based solution using the mouse monoclonal antibodies described here in the ICC assay.

As another example to demonstrate the ICC assay described herein can be applied to detect cervical cancer cells in liquid based solution, different carcinoma of cervical scrape samples in different liquid based solution were also prepared to perform ICC assay described in this invention. Figure 8 shows cervical cancer cells (diagnosed as adenocarcinoma by papanicolaou staining) can be ICC stained positively using an anti-E6 monoclonal antibody. In Figure 8, the abnormal cell with high N/C (nuclear/cytoplasm) ratio (indicated as black arrow) was stained positively. HSIL abnormal cells in the form of connecting each other were also stained positively. These results demonstrate that HPV E6 protein present in situ can be detected in adenocarcinoma cervical cells in liquid based solution using the mouse monoclonal anti-HPV E6 described here in the ICC assay.

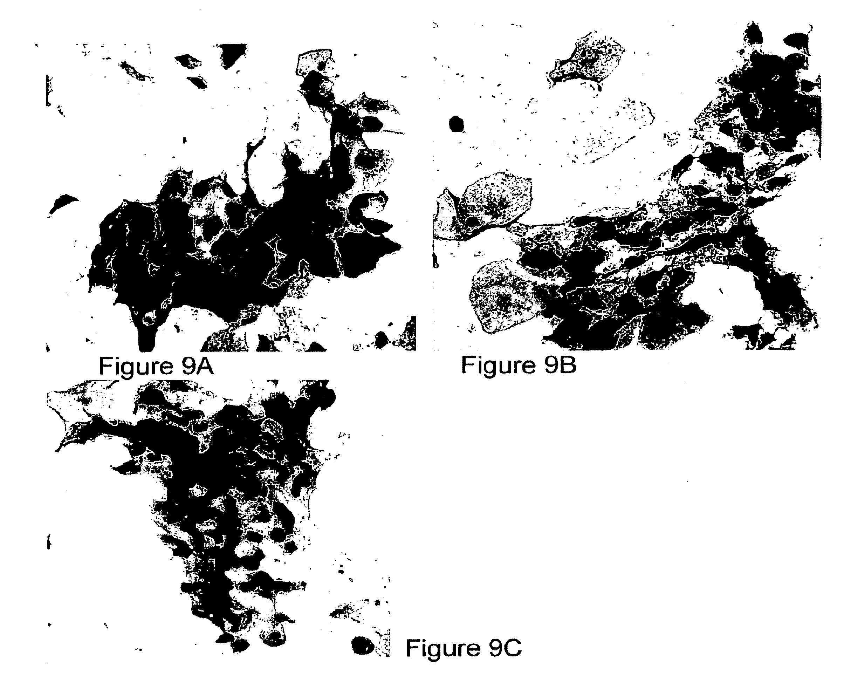

To demonstrate the ICC assay described herein can also be applied to detect another type of cervical cancer cells in liquid based solution, Figure 9A shows another type of cervical cancer cells, SCC (diagnosed as squamous cell carcinoma by papanicolaou staining) can be ICC stained positively using an anti-E6 monoclonal antibody. Figure 9B shows the results of ICC staining of the same SCC sample using an anti-HPV E7 mouse monoclonal antibody. Figure 9C shows the results of ICC staining of the same SCC sample using an anti-p16 mouse monoclonal antibody. As Figures shown, the HSIL SCC cells were stained positively to the nucleus and cytoplasm. These results demonstrate that HPV E6 and HPV E7 protein present in situ can be detected from various type of cervical cancer cells in liquid based solution using the mouse monoclonal anti-HPV E6 or anti-HPV E7 antibodies described here in the ICC assay. To confirm p16 is also overexpressed in the late stage of neoplasm, the same SCC sample was used to do ICC staining using an anti-p16 mouse monoclonal antibody. Figure 9C shows the results of ICC staining of the same SGC sample shown in Figure 9A-9B using an anti-p16 mouse monoclonal antibody, indicating that p16 protein present in situ can be detected in the late stage of neoplasm. These results demonstrate that HPV E6, HPV E7, and p16 protein can be detected in situ in the abnormal cells from different type of cervical cancers of liquid based solution using the mouse monoclonal antibodies described here in the ICC assay.

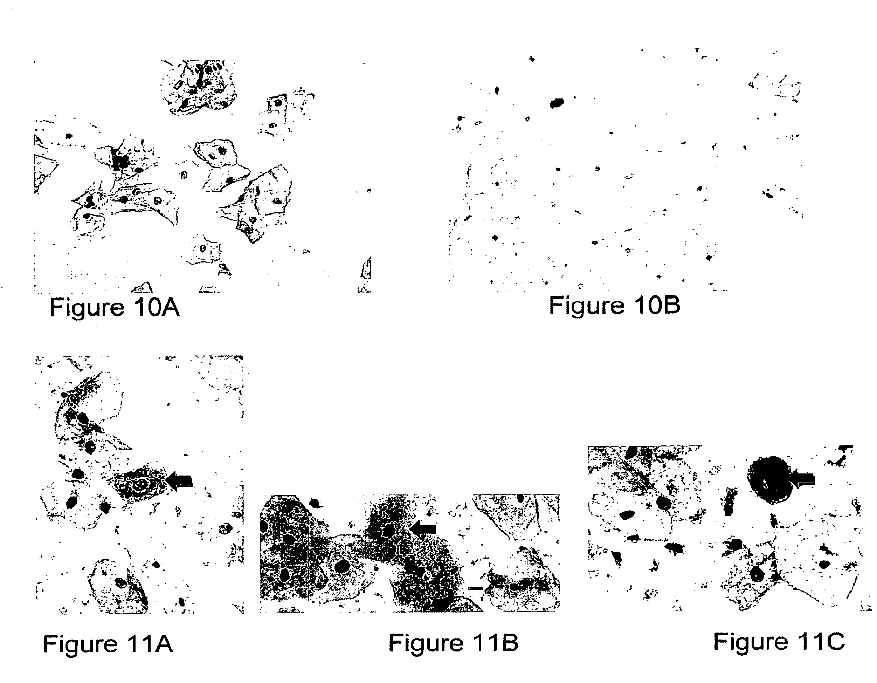

To confirm the ICC staining results described herein are specific binding of the HPV antibody with the HPV proteins present in situ of the cervical scrape cells, normal cervical cells from liquid based solution were also obtained to test on the ICC assay. Figure 10A shows all cervical scrape cells (diagnosed as normal by papanicolaou staining) prepared in liquid base solutions stained negatively by ICC using an anti-E6 monoclonal antibody. The same samples were also stained by ICC using an anti-HPV E7 monoclonal antibody as shown in Figure 10B. These results demonstrate that neither HPV E6 protein, nor HPV E7 protein present in situ from the normal cervical scrape cells, thus the ICC assay shows negative staining results using the mouse monoclonal anti-HPV E6 or the mouse monoclonal anti-HPV E7 antibody. Therefore, the ICC assay described in this invention is specific staining method for detection of HPV proteins using the HPV specific antibodies described herein.

To demonstrate the ICC staining at cellular level, Figure 11A shows cytoplasmic staining of cervical scrape cells from liquid based solution using a mouse monoclonal Anti-HPV-E6 antibody. Figure 7B shows nuclear staining of the same sample from Figure 11A staining by a mouse monoclonal anti-HPV-E7. Figure 11C shows representative images of cytoplasmic staining from another sample using a mouse monoclonal anti-HPV-E7 antibody. As shown in the figures, the abnormal cell with high N/C (nuclear/cytoplasm) ratio (indicated as black arrow) was stained positively while the normal cells (big, irregular cell shape with small nuclear) stain negatively as indicated by the white arrow. These results indicate that HPV E6 and HPV E7 proteins can be detected in cytoplasm and/or nuclear using the mouse monoclonal anit-HPV E6 and the mouse monoclonal anti-HPV E7 antibody described herein.

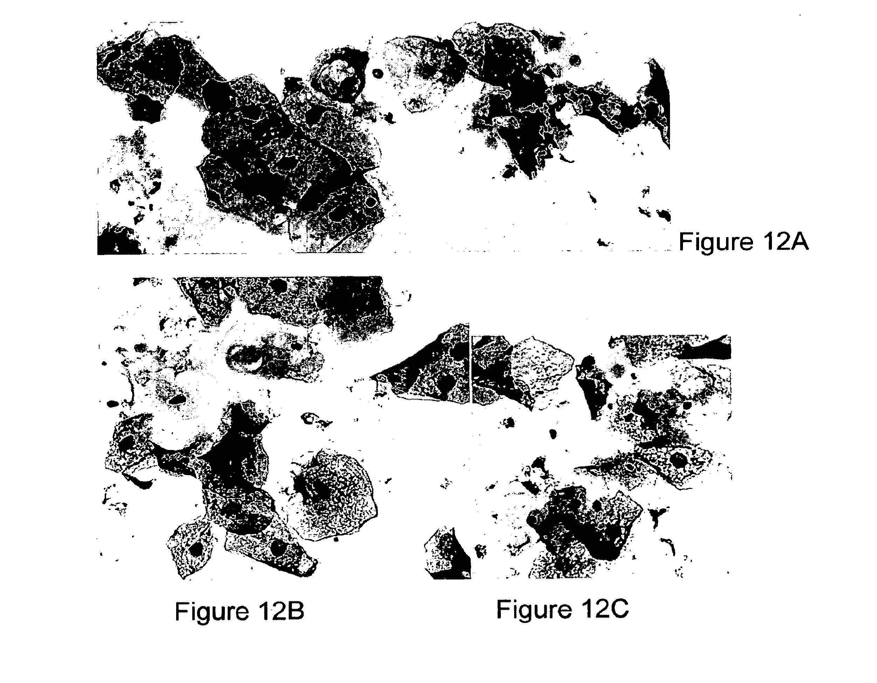

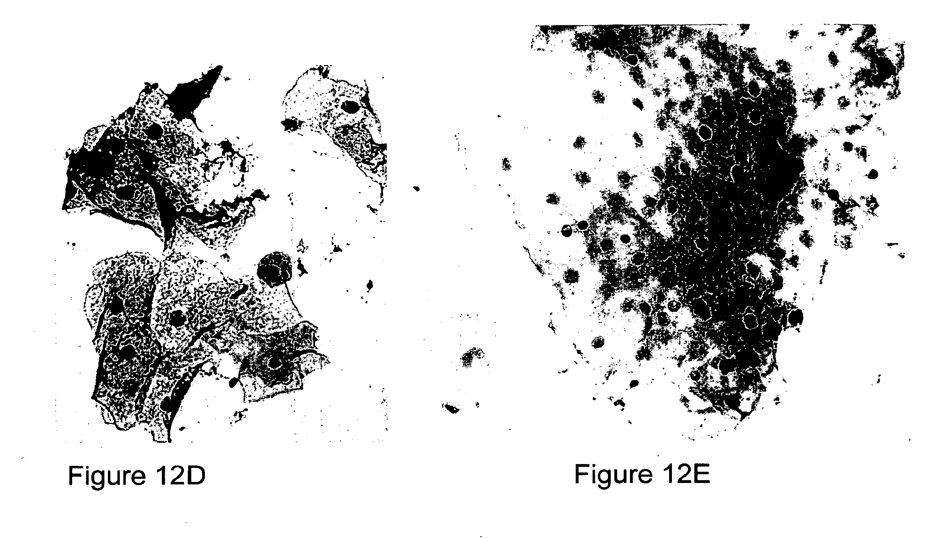

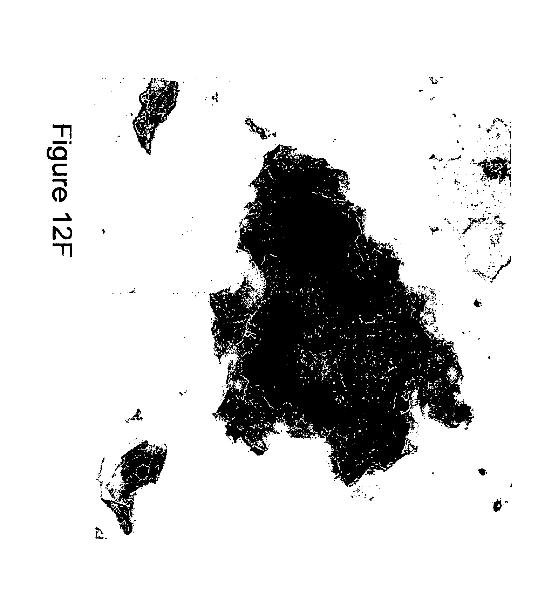

To demonstrate HPV E6 E7 oncoproteins can be expressed in certain LSIL (Low grade of Squamous Intraepithelial Lesion) or CIN 1 (Cervical Intraepithelial Neoplasia, mild cell abnormalities), Figure 12A-12C shows the results of ICC staining of a clinical sample diagnosed as CIN1 in a liquid based solution using an anti-HPV E6 mouse monoclonal antibody. Figure 12B-12C shows another image of the same ICC staining results shown in Figre 12A using the same anti-HPV E6 mouse monoclonal antibody. Figure 12D shows the results of of ICC staining from the same CIN1 sample shown in Figre 12A using an anti-HPV E7 mouse monoclonal antibody. Figure 12E shows another image of the same ICC staining results shown in Figre 12D using the same anti-HPV E7 mouse monoclonal antibody. As Figures shown, the abnormal, LSIL, CIN1 cells in the form of connecting each other or with high N/C (nuclear/cytoplasm) ratio (indicated as black arrow) was stained positively to the nucleus and cytoplasm. These results demonstrate that HPV E6 and HPV E7 protein present in situ can be detected in early stage of neoplasia in liquid based solution using the mouse monoclonal anti-HPV E6 or anti-HPV E7 antibodies described here in the ICC assay. To confirm if p16 is expressed in the early stage of neoplasia, the same CIN1 sample was used to do ICC staining using an anti-p16 mouse monoclonal antibody. Figure 12F shows the results of ICC staining from the same CIN1 sample shown in Figre 12A-12E using an anti-p16 mouse monoclonal antibody, indicating that p16 protein can not be detected in the early neoplasia of this CIN1 sample. These results demonstrate that HPV E6, HPV E7 proteins present in situ can be detected in the abnormal cells from early stage of neoplasia using the mouse anti-HPV E6, or anti-HPV E7 monoclaonal antibodies described in this invention, but p16 is not detectable by the anti-p16 mouse monoclonal antibody using the ICC assay described herein.

An immunocytochemical (ICC) assay not only detects HPV infection, but also detects HPV oncogenic proteins *in situ.* Therefore, ICC assay alone, or in combination with various specific and common anti-HPV antibodies can be a powerful tool for HPV detection *in situ,* as compared to a standard HPV DNA test or pap smear assay.

**Table 15: ICC staining results using a mouse anti-HPVE6 monoclonal antibody on various cervical scrape samples in a liquid based solution.**

| Pap smear | normal | ASCUS | ASC-H | CIN1 | CIN2/3 | SCC |
|---|---|---|---|---|---|---|
| ICC positive, using an anti-HPV E6 antibody | 4 | 3 | 4 | 11 | 17 | 4 |
| ICC negative, using an anti-HPV E6 antibody | 25 | 6 | 4 | 6 | 0 | 1 |
| total | 29 | 9 | 8 | 17 | 17 | 5 |
| positive rate | 14% | 33% | 38% | 65% | 100% | 80% |

Table 15 shows the results of an ICC assay using a mouse anti-HPVE6 monoclonal antibody on various cervical scrape samples in a liquid based solution. The results in Table 15 demonstrate that HPV E6 protein can be detected *in situ* on single cells fixed on a slide by immunocytochimical (ICC) assay using a mouse monoclonal anti-HPV E6 antibody. The *in situ* presence of HPV E6 proteins can be detected from various stages of cervical scrape samples in various liquid based solutions. The same cervical scrape samples were also processed by standard papinouli staining to compare the ICC staining results with the pap smear results. As shown in Table 15, HPV E6 proteins are present in the cervical scrape normal, ASCUS, ASC-H, CIN1, CIN2/3 samples with increasing positivity rate, respectively.

There is about 100% positive rate for samples diagnosed with pap smear CIN2/3, while only 14% of samples diagnosed with pap smear normal stained positively by ICC using the same anti-HPVE6 antibody. For ASCUS or ASC-H samples, about 33% to 50% of these samples are stained positively by the same anti-HPV E6 antibody as used for the CIN1, CIN2/3 samples shown in Table 15, indicating expression of oncogenic proteins in these ASCUS or ASC-H sample subjects to be followed up for further cancer progression. For samples with pap smear diagnosed as ASCUS and ICC staining (anti-HPV E6) as negative, it may have less risk to develop progressive lesion.

**Table 16: Summary of the ICC staining results using a mouse monoclonal anti-HPVE6 antibody on CIN2+ cervical scrape samples in a liquid based solution.**

| Pap smear | normal | CIN2+ | | |
|---|---|---|---|---|
| ICC positive, using an anti-HPV E6 antibody | 4 | 21 | 84% | PPV |
| ICC negative, using an anti-HPV E6 antibody | 25 | 1 | 96% | NPV |
| Sensitivity | | 95% | | |
| specificity | 86% | | | |

**Table 17: ICC staining results using a mouse monoclonal anti-HPVE7 antibody on various cervical scrape samples in a liquid based solution.**

| Pap smear | normal | ASCUS | ASC-H | CIN1 | CIN2/3 | SCC | |
|---|---|---|---|---|---|---|---|
| ICC positive, using an anti-HPV E7 antibody | 3 | 4 | 3 | 11 | 16 | 4 | |
| ICC negative, using an anti-HPV E7 antibody | 25 | 6 | 5 | 6 | 1 | 1 | |
| Total | 28 | 10 | 8 | 17 | 17 | 5 | |
| positive rate | 11% | 40% | 38% | 65% | 94% | 80% | |

Table 16 shows summary of the ICC staining results from Table 15. As data indicated, the ICC staining method using the anti-HPV E6 antibody described in this invention provides ICC assay sensitivity of 95% for CIN2+ with specificity of 83%. These data suggest this assay can be useful to detect HPV proteins for screening of cervical cancer from general population along with routine pap smear staining. As another example of the HPV detecting ICC assay, Table 17 and Table 18 show results of ICC staining using anti-HPV E7 antibody. As data shown, HPV anti-E7 gives comparable ICC results as HPV anti-E6 shown. Table 17 shows that HPV E7 proteins are present in the cervical scrape normal, ASCUS, ASC-H, CIN1, CIN2/3 samples with increasing positivity rate, respectively. There is about 94% positive rate for samples diagnosed with pap smear CIN2/3, while only 11% of samples diagnosed with pap smear normal stained positively by ICC using the same anti-HPVE7 antibody. For ASCUS or ASC-H samples, about 40% of these samples are stained positively by the same anti-HPV E7 antibody as used for the CIN1, CIN2/3 samples shown in Table 17, indicating expression of oncogenic proteins in these ASCUS or ASC-H sample subjects to be followed up for further cancer progression. For samples with pap smear diagnosed as ASCUS and ICC staining (anti-HPV E7) as negative, it may have less risk to develop progressive lesion. Table 18 shows summary of the ICC staining results from Table 17. As data indicated, the ICC staining method using the anti-HPV E7 antibody described in this invention provides ICC assay sensitivity of 91% for CIN2+ with specificity of 89%. These data suggest this assay can be useful to detect HPV proteins for screening of cervical cancer from general population along with routine pap smear staining.

**Table 18: Summary of the ICC staining results using a mouse monoclonal anti-HPVE7 antibody on CIN2+ cervical scrape samples in a liquid based solution.**

| Pap Smear | Normal | CIN2+ | | |
|---|---|---|---|---|
| ICC positive, using an anti-HPV E7 antibody | 3 | 20 | 87% | PPV |
| ICC positive, using an anti-HPV E7 antibody | 25 | 2 | 93% | NPV |
| Sensitivity | | 91% | | |
| specificity | 89% | | | |

**Table 19: ICC staining results for pap smear normal samples using various anti-HPV antibodies compared to HPV DNA test**

| | Pap smear normal | |
|---|---|---|
| | ICC HPV positive | ICC HPV negative |
| high-grade HPV DNA positive | 0 | 6 |
| high-grade HPV DNA negative | 0 | 26 |

To test if the HPV ICC assay described in this invention is suitable for early stage of cervical cancer screening, pap smear normal samples are used to compare the HPV ICC assay with HPV DNA test. As data shown in Table 19, all pap smear normal samples tested (32 out of 32) stain negatively using anti-HPV antibody. Among the 32 samples, 12 samples were stained with anti-HPV E6 antibody, 16 samples were stained with anti HPV E7 antibody, and 4 samples were stained with anti-HPV L1 antibody. These data indicate that the ICC staining assay described in this invention is very specific. Comparing to HPV DNA test results on the same samples, 19% (6 out of 32) of the pap smear normal samples show positively on HPV DNA test. The high-grade HPV DNA test used in this study was hc2, the only FDA approved HPV DNA test. For those HPV DNA positive, pap smear normal and HPV ICC negative samples, it is possible false positive of the HPV DNA assay, or the DNA detection of HPV infection with no expression of HPV oncogenic proteins. These data indicate that HPV ICC assay described herein provides better positive predictive value compared to HPV DNA test, thus provides better clinical relevance for screening of cervical cancer.

**6. HPV Flow cytometry assay.** As another example of immunoassay for detection of HPV proteins, HPV flow cytometry assay is performed. Cells from cervical scrapes collected in liquid based solution following manufacture guideline were centrifuged, washed, immunostained following the similar ICC staining procedure. Instead of applying cells onto a slide, the cells are kept in solution from staining through analysis by flow cytometry. To perform HPV flow assays, the cervical cells in solution were fixed, blocked and incubated with anti-HPV antibodies followed by appropriate secondary antibody and substrate agents used for detection by flow cytometry. The advantage of this HPV flow cytometry assay is high throughput with no requirment for a highly trained cytologist to view the slides.

In one embodiment, a kit for performing an ICC flow cytometry assay is provided. The kit may include a pre-antibody blocking solution, post-antibody blocking solution, an anti-HPV antibody as the primary antibody, an anti-mouse or anti-rabbit immunoglobulins conjugated with flourecent or biotin, or other agents as secondary antibody, a solution containing appropriate agents used as substrate for the secondary antibody to be detected by flow cytometry.

As an example, indirect labelling requires two incubation steps; the first with a primary antibody followed by a compatible secondary antibody. The secondary antibodies have the fluorescent dye (FITC, PE, Cy5, etc.) conjugated. The anti-HPV antibodies may also be directly tagged with fluorescent, or biotin, or other agents to be detected following appropriate agents used as substrate. The pre-antibody blocking solution may contain certain proteins or BSA, or serum or other agents to block the cells from nonspecific binding of antibody. The post blocking solution may contain similar solution as the pre-antibody blocking solution with less proteins or serum to be used along with primary antibody incubation. The solution containg HPV antibodies may be in concentrated form, or may be in diluted form as ready to use reagent. The solution containing secondary antibodies may be in concentrated form, or may be in diluted form as ready to use reagent.

As an example, the HPV E6, E7, or L1 protein can be detected by the specific antibody followed by the 2^{nd} antibody labeled with fluorescent dye (FITC, PE, Cy5, etc. conjugated). The cells analyzed by flow cytometry can be gated by size or other parameter to look at the cell population with or without staining. The stain intensity from cell population with smaller cell size can be compared to the cell population with bigger cell size as control of normal cells in the assay. This assay provides specific staining for individual cells. Number of cells stained or unstained can be counted, and the intensity of staining can also be quantitated by analysis. This assay can be high throughput, requires no microscope, nor cytologist to score the staining results. The powerful computer software from the flow cytometry provides all data for analysis with no bias or trained personel in cytology required. This assay should apply well in the clinical setting as screening test or accompanion test for detection of HPV associated proteins.

As another example, after the cells are stained, it is better to store the cell suspension immediately at 4°C in the dark and nalyze the cells on the flow cytometer as soon as possible. If longer than an hour before flow analysis, it's necessary to fix the cells. This can preserve them for at least several days. This will stabilize the light scatter and inactivate most biohazardous agents. The fixation for different antigens will require optimization for different assays. Format 1. Paraformaldehyde 0.01% to 1% for 10 -15 minutes only, 100µl per sample. Format 2: Acetone or methanol: Add 1ml ice cold acetone to each sample. Mix gently. Place at -20°C for 5 to 10 minutes. Centrifuge, wash twice in PBS 1% BSA

For intracellular staining, cells can be fixed first to ensure stability of soluble antigens or antigens with short half life. This should retain the target protein in the orginal cellular location. Detection of intracellular antigens requires a cell permeabilization step prior to staining. Antibodies should be prepared in permeabilisation buffer to ensure the cells remain permeable. When gating on cell populations, the light-scatter profiles of the cells on the flow cytometer will change considerably after permeabilization. Antigens in cytomplasmic organelles and granules will require a fixation and permeabilisation method depending on the antigen. The epitope needs to remain accessible.

Fixation could be critical for the quality of staining assay. There are several methods available for fixation: (1) Formaldeyde followed by detergent: Fixation in 0.01% formaldehyde for 10-15 minutes (this will stabilise proteins), followed by disruption of membrane by detergent. Detergents: Triton or NP-40 (0.1 to 1% in PBS). These will also partially dissolve the nuclear membrane and are therefore very suitable for nuclear antigen staining. It should be noted that loss of cell membrane and cytoplasm will result in decreased light scattering and also in reduced non-specific fluorescence. Tween 20, Saponin, Digitonin and Leucoperm are mild membrane solubilisers. Use at 0.5% in PBS. These give large enough pores for antibodies to go through without dissolving plasma membrane. Suitable for antigens in the cytoplasm or the cytoplasmic face of the plasma membrane. Also suitable for soluble nuclear antigens.(2) Formaldehyde (0.01%) followed by methanol. Methanol followed by detergent. Add 1ml ice cold methanol to each sample. Mix gently. Place at -20°C for 10 minutes. Centrifuge, wash twice in PBS 1% BSA. Acetone fixation and permeabilisation: Add 1 ml ice cold acetone to each sample. Mix gently. Place at -20°C for 5 to 10 minutes. Centrifuge, wash twice in PBS 1% BSA

### 7. Detecting HPV proteins on solid surface using one anti-HPV antibody.

1). Direct EIA: one or more HPV proteins coated on microtiterplate to be detected by one or more anti-HPV antibodies. Clinical samples from cervical scrapes were obtained for detection of HPV E6, E7 or L1 proteins on direct EIA. Cervical cells from various sample source include cervical scrape cells in liquid based cytology solution, cervical scrape cells in transport medium (used for HPV DNA test sample), or cervical scrape cells in lysis buffer. To perform the direct EIA described herein, specimens were processed, centrifuged, washed, and lysed to generate cell late as anyalyte. The proteins in the cell lysate was quantitated and coated to microtiterplate with the same amount of proteins for coating in each well. The plate was blocked, and detected by each HPV monoclonal antibody followed by HRP conjugated secondary antibody (anti-mouse IgG or anti-rabbit IgG for example). TMB substrate was added followed by a stopping solution. OD 450 was taken by an ELISA plate reader.

As an example, cervical cells from various stage of cervical neoplasm collected in liquid based solution are processed to obtain the cell lysate for detection of HPV DNA and HPV proteins. For HPV DNA detection, touchdown PCR protocol was used. For HPV protein detection by direct EIA, cell lysate was coated directly on the microtiterplate, blocked, then specific HPV antibodies were used followed by secondary antibody with HRP conjugated. OD450 was taken from microtiter plate coated with cell lysate with or without adding the primary HPV antibody followed by the secondary antibody. Since various proteins from the cell lysate was coated on the microplate, OD from each sample with no primary HPV antibody was considered as non-specific binding of the cell lysate with the secondary antibody. To obtain OD for specific binding of HPV protein with anti-HPV antibody, net OD for each sample obtained by subtraction of the OD from its non-specific binding of the secondary antibody was considered as specific binding of HPV protein with the primary anti-HPV antibody. Net OD from each PCR negative samples was obtained. Mean of OD from PCR negative samples was used as the baseline of the assay. Sample with net OD over two folds of average OD from PCR negative samples was considered positive, otherwise is negative for the EIA test described herein.

As an example, clinical samples diagnosed by histology or pap smear staining including abnormal cells, ASCUS, CIN1, CIN2, CIN3, SCC and Adenocarcinoma are obtained to process cell lysate for direct coating on microtiterplate. Compared to the clinical diagnosis or pap smear results, detection of HPV DNA by PCR and detection of HPV proteins by EIA are shown as Table 20. Data show 79% (19 out of 24) correlation of HPV DNA with HPV proteins detected by polyclonal anti-E7 antibody. For those HPV DNA and HPV proteins discrepency (21%; 5 out of 24), 3 (out of the 5) are PCR pos, EIA negative (case No.9/CIN1, No.12/CIN2, and No.13/CIN2) indicating HPV infection with no expression or non-detectable E7 oncoproteins. For those PCR negative, EIA positive like case No. 4 (ASCUS), and No. 10 (CIN1/ASCUS), it could be false negative of PCR, or E7 oncoproteins expressed with loss of HPV DNA.

**Table 20. Detection of HPV DNA and HPV protein from liquid based cervical scrapes**

| Liquid based cervical scrapes | | HPV DNA by | Direct EIA by |
|---|---|---|---|
| Sample No. | Dx or pap smear results | PCR | poly anti-E7 |
| 1 | ASCUS | positive | positive |
| 2 | ASC-H | positive | positive |
| 3 | ASCUS | positive | positive |
| 4 | ASCUS | negative | positive |
| 5 | ASCUS | negative | negative |
| 6 | CIN1 | positive | positive |
| 7 | CIN1 | positive | positive |
| 8 | CIN1 | positive | positive |
| 9 | CIN1 | positive | negative |
| 10 | CIN1/ASCUS | negative | positive |
| 11 | CIN1 | negative | negative |
| 12 | CIN2 | positive | negative |
| 13 | CIN2 | positive | negative |
| 14 | CIN2 | negative | negative |
| 15 | CIN2 | negative | negative |
| 16 | CIN3 | positive | positive |
| 17 | CIN3 | positive | positive |
| 18 | CIN3 | positive | positive |
| 19 | CIN3 | negative | negative |
| 20 | CIN3 | negative | negative |
| 21 | CIN3 | negative | negative |
| 22 | SCC | positive | positive |
| 23 | SCC | positive | positive |
| 24 | AD | negative | negative |

For those HPV DNA positive but HPV EIA negative samples, it is possible false positive of the HPV DNA assay, or positive HPV DNA detection with no expression of HPV oncogenic proteins. These data indicate that HPV EIA described herein provides additional clinical relevance for screening of cervical cancer. It's important to detect the HPV oncoporteins to follow up if progression of dysplasia HSIL occurs. These data indicate that the HPV oncoproteins are good biomarkers for screening and early detection of cervical cancers, and other HPV associated cancers. For both PCR and EIA negative but diagnosed ASCUS, or CIN cases, the loss of HPV DNA and HPV oncoprotein detection is possibly due to the sampling of cervical scrapes cells or treatment of the patients, or false positive of the pap smear results. However, more samples to be tested are mandatory.

### 2). Dot Blot assay: spotting cell lysate on membrane to detect HPV proteins from biological samples using one or more anti-HPV antibodies

To develop a rapid test showing results with no instrument required for the read out, dot blot assay demonstrates the feasibility of detecting HPV proteins from cell lysate on a membrane with visual results followed by colormetric substrate. As an example, cervical cells from various stage of cervical neoplasm collected in liquid based solution are processed to obtain the cell lysate to be spotted on a membrane. The membrane was air dry prior to blocking the blot with blocking solution. An anti-HPV antibody was added to react with the blot followed by a secondary antibody capable of binding to the anti-HPV antibody. The blot was washed between each step to avoid non-specific binding of the antibody onto the membrane. In the final step, TMB substrate was added to the blot showing blue spot as positive reaction of the cell lysate binding with the anti-HPV antibody used in this dot blot assay. Recombinant HPV proteins were also spotted on the membrane to be used as positive or negative control

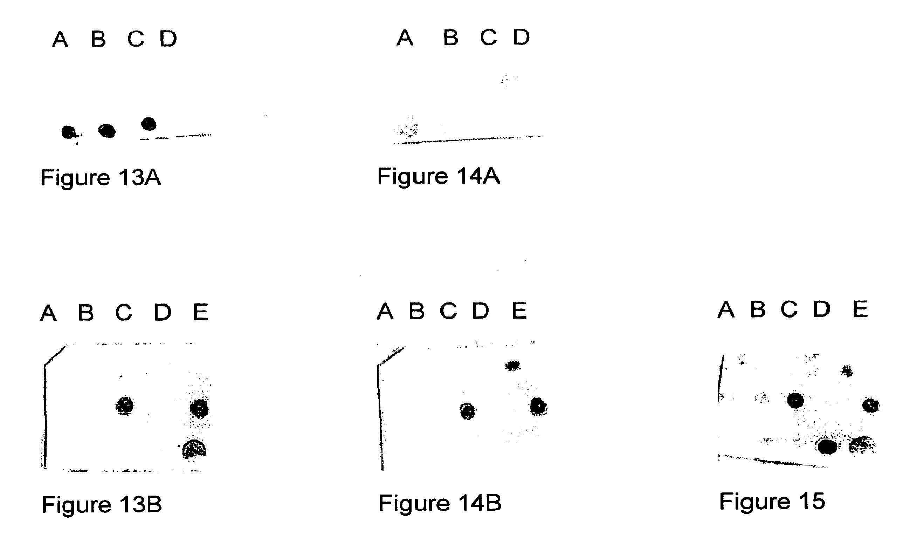

Figure 13A shows results of a dot blot detecting HPV L1 proteins using a mouse monoclonal anti-HPV L1 antibody. As indicated, dots from ^{1st} row are cell lysate from various SCC cervical scrapes in liquid based solution and dots from 2^{nd} row are recombinant HPV16 L1 protein at concentration of 20, 2, 0.2 and 0 ug/ml from left to the right as indicated A, B, C, D respectively. As the results indicated in the 2^{nd} row of the blot, recombinant HPV L1 proteins reacts highly positive to 0.2 ug/ml or lower of purified recombinant proteins with the mouse monoclonal anti-HPV L1 antibody used in Figure 13A. These data demonstrate that HPV L1 proteins from both recombinant HPV 16 L1 and cell lysate can be detected by dot blot assay using a mouse monoclonal anti-HPV L1 antibody as shown in Figure 13A.

Figure 13B show results of another dot blot detecting HPV L1 proteins using the same mouse monoclonal anti-HPV L1 antibody shown in Figure 13A. As indicated, dots from ^{1st} and ^{2nd} row are cell lysate from various SCC cervical scrapes in liquid based solution. In the 3rd row of the blot, recombinant HPV16 E6, HPV18 E6, HPV16 E7, HPV18 E7, HPV16 L1 proteins are spotted from left to right as indicated A , B, C, D,E respectively. As the results indicated in the 3^{rd} row of the blot, recombinant HPV L1 proteins, spot 3E, reacts positively with the mouse monoclonal anti-HPV L1 antibody used in the assay, compared to HPV16 E7 and HPV16 E6 with no detectable spot or HPV18 E7 and HPV18 E6 with very weak spot shown on the 3^{rd} row of the blot. Using those weak spot shown as background or non-specific binding of the assay, clinical sample 2C, and 2E show spots as strong as HPV16 L1 recombinant proteins. Figure 13A indicate that HPV L1 proteins from both recombinant HPV 16 L1 and cell lysate can be detected by dot blot assay using a mouse monoclonal anti-HPV L1 antibody.

To detect HPV E6 protein on dot blot assay, Figure 14A shows results of a dot blot with a mouse monoclonal anti-HPV E6 antibody. As indicated, dots from ^{1st} row are cell lysate from various SCC cervical scrapes (same as the first row of Figure 1) in liquid based solution and dots from 2^{nd} row are recombinant HPV16 E6 protein at concentration of 20, 2, 0.2 and 0 ug/ml from left to the right as indicated A, B, C, D respectively. As the results indicated in the 2^{nd} row of the blot, recombinant HPV E6 proteins reacts positively to 20, and 2 ug/ml, and weakly to 0.2 ug/ml or lower of purified recombinant proteins with the mouse monoclonal anti-HPV E6 antibody used in Figure 3. These data indicate that HPV E6 proteins from both recombinant HPV 16 E6 and cell lysate can be detected by dot blot assay using a mouse monoclonal anti-HPV E6 antibody demonstrated in Figure 14A.

The same spotting blot shown in Figure 13B was also used to demonstrate detection of HPV E6 protein on dot blot assay. Figure 14B shows results of a dot blot detecting HPV E6 proteins using the same mouse monoclonal anti-HPV E6 antibody shown in Figure 14A. As the results indicated in the 3^{rd} row of the blot, recombinant HPV16 E6 proteins, spot 3A reacts positively with the mouse monoclonal anti-HPV16 E6 antibody used in the assay, compared to HPV 18 E6 with very weak spot and other recombinant proteins with no detectable spots shown on the 3^{rd} row of Figure 13B. These results demonstrate specific reaction of HPV E6 proteins with the mouse monoclonal anti-HPV E6 antibody, not cross reacting with HPV L1, or HPV E7 proteins. Using the weak spot shown as background or cross-reactive binding of HPV 18 E6 in the assay, sample 2C and 2E shows very strong spots compared to others moderate spots and 2D with no detectable spot. These data indicate 70% (7 out of 10) clinical samples containing HPV E6 proteins can be detected by dot blot assay using a mouse monoclonal anti-HPV16 E6 antibody demonstrated in Figure 14B.

To demonstrate detection of HPV E7 proteins on dot blot assay, the same spotting blot shown in Figure 13B and Figure 14B was also used to blot with a mouse monoclonal anti-HPV E7 antibody detecting HPV E7 proteins as shown in Figure 15. As the results indicated in the 3^{rd} row of the blot in Figure 15, recombinant HPV18 E7 proteins, spot 3D reacts positively with the mouse monoclonal anti-HPV18 E7 antibody used in the assay, compared to other HPV recombinant proteins with no detectable spots, or a very weak spot with HPV16 L1, spot 3E, shown on the 3^{rd} row of Figure 15. These results demonstrate specific reaction of HPV E7 proteins with the mouse monoclonal anti-HPV E7 antibody, not cross react with HPV L1, or HPV E7 proteins. Using the weak spot shown as background or cross-reactive binding of HPV 18 E7 in the assay, sample 2C and 2E shows very strong spots compared to others with no detectable spots. These data indicate sample 2C and 2E containing HPV18 E7 proteins can be detected by dot blot assay using a mouse monoclonal anti-HPV18 E7 antibody demonstrated in Figure 15.

### 3). Antibody microarray: spotting antibodies on a protein chip to detect HPV proteins and cellular endogenous proteins in a labeled cell lysate from biological sample

For example, in a protein chip assay, the surface for proteins to be coated/bound to may be, for example, a surface-chemistry treated glass or membrane, which can be covalently or non-covalently bind or coat with capture agents or proteins thereto. A spotting machine with fine pins dipped with capture agents, such as the recombinant proteins, antigens, antibodies, or other proteins, in suitable buffers is generally used to facilitate binding of such proteins or antibodies to the treated surface. Like other surfaces described in the microtiter plate format, the spotted and thus captured proteins or antibodies bind strongly to the surface-chemistry treated surface of a protein chip and remain on the treated surface to allow the interaction and specific binding of the captures proteins with target proteins, antibodies, or antigens, even after several washings of removing non-specific binding, to be detected with a detection system having conjugated with Cy3 or Cy5. The detection of specific interaction is obtained and measured by the fluorescent intensities of the spotted/dipped images via a microarray scanner.

As an example, antibody microarray can be used as the protein chips assay format for detection of HPV protein and other cellular proteins. First, cells, samples or cultured cells to be tested were collected, centrifuged, washed, and lysed to generate cell late as anyalyte. The protein in the cell lysate was quantitated and labeled with biotin or Cy3, Cy-5 or any other chromagen for subsequent detection of binding of such labeled protein on the surface of prespotted antibody. The surface of the protein chips for the protein chip assays can be a membrane or glass for different analysis and quantification techniques.

Table 21 shows the results of protein chip assays for detecting the presence of various HPV proteins and various host cellular proteins. An antibody array prespotted with antibodies against HPV and various cellular proteins were used to detect the presence of these HPV proteins and host cellular proteins in a human cervical scrape clinial sample. Total of 10 samples of cervical scrapes (labeled as S1-S10 shown on table 2) diagnosed as keratinizing squamous cell carcinoma (grade 2 or grade 3) obtained from liquid based solution was processed and lysed to generate protein lysate with proper labeling (such as biotin label) for subsequent detection (followed by strepavidin-Cy3, for examples). The fluorescent intensity indicating binding of the proteins from the human sample with prespotted antibody against proteins included, but not limited, such as HPV-16 E7, HPV-16 L1, p63, p53, p21WAF1, p16INK4a, phosphorylated Rb, and unphosphorylated Rb was obtained and shown in Table 21. Flourescent intensity for binding of the specific protein with the specific antibody prespotted on the microarray, indicating changes in the expression levels of these proteins affected by HPV infection was also compared and analyzed to be shown in Figure 6 to Figure 12.

**Table 21: Flourescent intensity (after background subtraction) of individual spot shows binding of specific antibody with proteins expressed in cell lysate from cervical cancer patients on antibody microarray**

| Ab spotted | S1 | S2 | S3 | S4 | S5 | S6 | S7 | S8 | S9 | S10 |
|---|---|---|---|---|---|---|---|---|---|---|
| HPV16 E7 | 849 | 1407 | 422 | 355 | 443 | 403 | 316 | 337 | 383 | 267 |
| HPV 16 | 1309 | 236 | 1477 | 418 | 620 | 1206 | 251 | 205 | 700 | 3407 |
| p63 | 398 | 128 | 205 | 51 | 167 | 146 | 215 | 230 | 427 | 174 |
| p53 | 325 | 102 | 86 | 161 | 119 | 83 | 226 | 242 | 465 | 335 |
| p21WAF1 | 594 | 100 | 130 | 92 | 167 | 54 | 177 | 178 | 493 | 250 |
| p16INK4a | 164 | 549 | 97 | 107 | 116 | 87 | 72 | 128 | 87 | 174 |
| Retinoblastoma | 753 | 170 | 140 | 185 | 109 | 70 | 219 | 247 | 448 | 317 |
| Rb (phosph) | 491 | 236 | 269 | 143 | 238 | 245 | 156 | 224 | 310 | 171 |

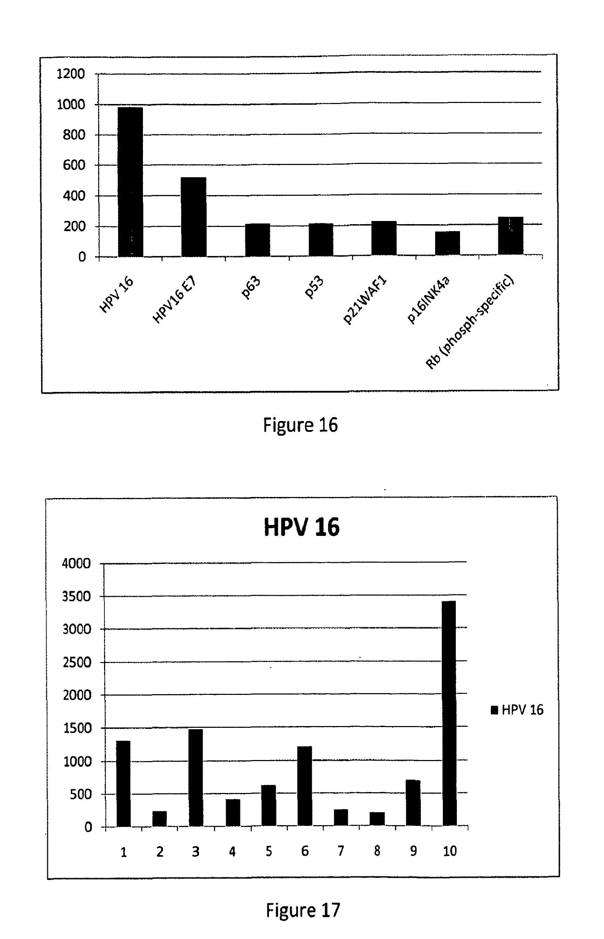

To compare the expression of each protein from the 10 SCC samples tested, average of fluorescent intensity for a specific protein from each sample was obtained to demonstrate the the protein expression level with standard deviation bar for the graphs show in Figure 16. Results indicate various HPV proteins and various cellular endogenous proteins from cell lysate of the 10 cervical scrape samples can be detected on the antibody microarray assay described herein. As indicated in Figure 16, HPV 16 and HPV16E7 are over expressed comparing to other cellular proteins.

To demonstrate variation of HPV16 in different sample, Figure 17 shows flourescent intensity of each sample for detecting HPV L1 proteins in cell lysate from cervical scrape cells shown in Figure 16. The results demonstrate binding of HPV16 antibody (anti-HPV L1 antibody) with HPV proteins, particular the L1 viral protein expressed in cell lysate from cervical cancer patients on antibody microarray. HPV16 L1 proteins express predominantly in sample S1, S3, S6, and S10, medium for sample S4, S5, and S9 while express low in sample S2, S7, and S8 which might be different type of HPV not recognized by the HPV16 antibody used in this assay.

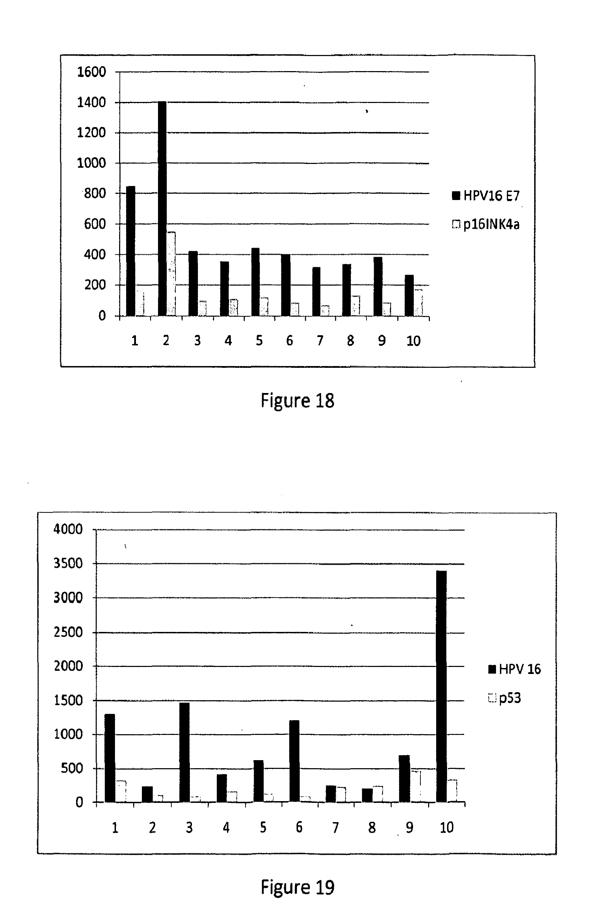

HPV E6 and E7 proteins play critical roles in oncogenesis of HPV in cervical cancer. To study the interaction of the HPV E6 E7 oncoproteins with cellular proteins, the antibody microarray assay described herein provides tools for simultaneous detection of HPV proteins and cellular proteins such as p53, or Rb which directly interacted with HPV oncoproteins or cellular proteins such as p16, p21, etc affected by HPV infection. P16INK4a has been commonly used as a surrogate for detection of cervical cancer. To demonstrate HPV viral proteins, such as E6 or E7 oncoprotein can serve as a better biomarker for detecting cervical cancer, the antibody microarray (protein chip assay) described herein demonstrate detection of multiple proteins including various HPV proteins and various cellular proteins simultaneously. Figure 18 shows detection and comparision of HPV E7 and p16 protein expression in the 10 SCC samples. Flourescent intensity from each individual sample (1 through 10) demonstrates binding of HPV16E7 antibody and p16INK4a antibody with proteins expressed in cell lysate from cervical cancer patients on antibody microarray. For the 10 samples test, each sample shows higher fluorescent intensity with HPV E7 antibody than p16 antibody, indicating more HPVE7 protein expressed compared to p16. These data suggesting HPVE7 serve as a better marker for detecting cervical cancer.

To demonstrate p53 affected by HPV in cervical cacner, Figure 19 shows fluorescent intensity for both HPV16 and p53 antibody, indicating overexpression of HPV16 with p53 suppression in clinical smaples with HPV infection. Comparing expression of HPV16 and p53, the results indicate p53 is expressed at much lower level with high level of HPV16 expression in most clinical samples except clinical samples S7 and S8, which might be different type of HPV other than HPV 16 infection. However, low p53 expression in all clinical samples indicates most p53 proteins are degraded by HPV E6 oncoproteins during transformation of cervical cancer development

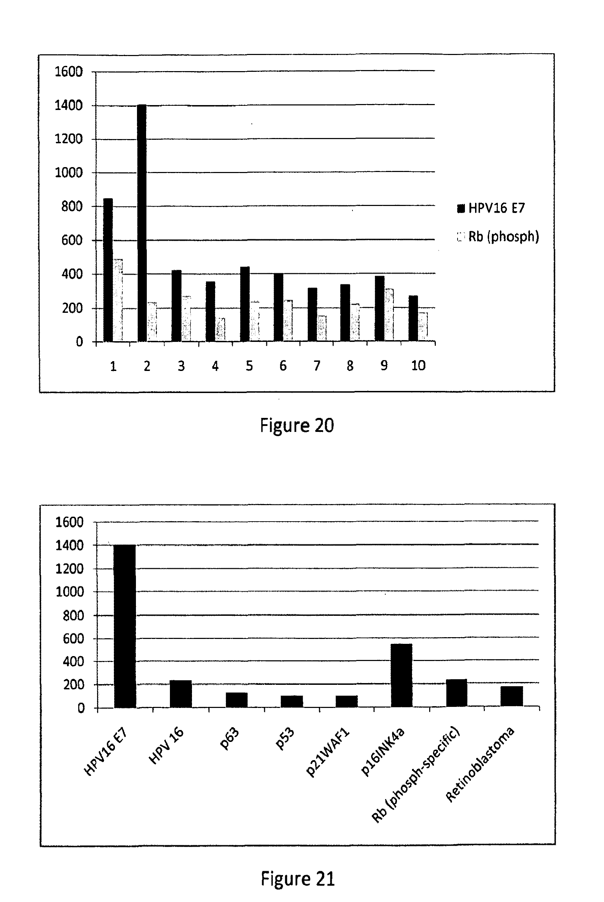

To demonstrate interaction of E7 and retinoblastoma (Rb) protein and phosphorylated Rb affected by HPV in cervical cancer, Figure 20 shows fluorescent intensity for HPV16E7 and pRb antibody, indicating HPV16 E7 expression at higher level while Rb is inactivated, which is recognized by anti-Rb-phosphate specific antibody at lower level. Comparing HPV16 E7 expression over Rb-phosphate, overexpression of HPV16 E7 with phosphrylated Rb suppression is predominant in sample S2. Data indicate inactivation of Rb (low in reacting with Rb-phosphate antibody) by E7 pathway cause malignant transformation developing cervical cancer.

To demonstrate expression profiling of protein chip assay for cervical cancer as an example, Figure 21 shows expression profiling of the setected HPV proteins and cellular proteins for sample S2. Results indicate HPV E7 and p16 was overexpressed, while other cellular proteins are suppressed. All together with results from Figure8, Figure 20, and Figure 21 suggest overexpression of HPV E7 protein in sample #2 inactivates Rb and induces p16INK4a expression, resulting in malignant transformation and the development of cervical cancer. Sample S1 with high HPV-E7 expression level may undergo pathway independent from Rb, thus didn't express high level of p16INK4a.

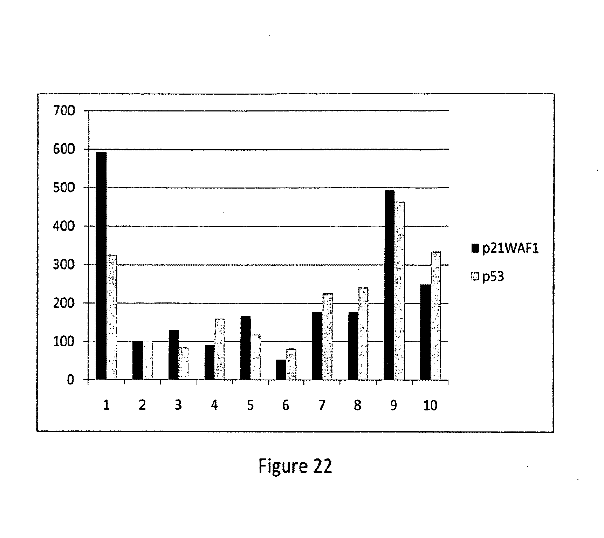

To demonstrate another cellular protein p21 WAF1 expression in cervical cancer and its correlation with p53, Figure 12 shows flourescent intensity of each sample for detecting cellular p21 WAF1 and p53 proteins using cell lysate from cervical scrape cells. As data shown in Figure 22, there is 9 out of 10 samples (except sample S1) expressing p21 WAF1 well correlated with expression of p53. These data demonstrate degradation of tumor suppressor p53 by HPV-E6 pathway through p21WAF1 inhibition causes malignant transformation for cervical cancer development.

Protein chip assays provide tools to detect HPV proteins as well as cellular protein induced and/or inhibited by HPV infection in malignant of cancer development. Study results using protein chip assay described in the invention indicate that patients with cervical cancer in this study adopt various pathways, thus progress differently. This technology applies to other HPV associated cancers to predict the pathways involved in malignance. An algorisum can be developed to predict the signature of all proteins involved in the pathways during cancer development. Thus recommendation of specific treatment for personalized medicine can be provided.

### 8. Detecting HPV proteins from biological samples using first anti-HPV antibody captured on a surface to be detected by a second anti-HPV antibody

As an example, an antigen sandwich assay involves coating of a first antibody, such as a capture antibody or a spotting antibody, having an affinity for binding to an antigen of interest, on a surface, such as bottom surfaces of a protein chip, a membrane and/or a microtiter plate, *etc.* The antigen of interest may be, for example, a papillomarivus protein, an oncoprotein, a capsid protein, which may be encoded by a HPV viral gene, e.g., an early gene or a late gene, *etc.* After blocking unbound portions on the surface, the clinical sample to be analyzed can be applied to bind with the capture antibody to form an immunocomplex, which can be detected by a second antibody or a detection antibody by binding to the antigen of interest. Hence, the first and the second antibodies or the pair of the capture antibody and the detection antibody interact with the antigen of interest, much like a sandwich. The capture or spotting antibody can be the same or different antibody as the detection antibody as long as the two antibodies can specifically bind to the antigen of interest, e.g., a HPV viral protein, a HPV oncoprotein, a capsid protein, among others.

Next, the sandwiched bound antibody-antigen complex can be detected by a secondary antibody, which have an affinity for the detection antibody and facilitate measurement by a standard immunological complex detection system using colormetric, chemilumenescent, flourescent and many different kinds of substrates. The final readouts or visualizations can be performed by an instrument with appropriate light absorbance readers or directly visualized by eye and compared the results to a control sample. Positive results indicate binding of the antigen of the interest to the primary antibodies, the capture antibody, and the detection antibody, and thus the presence of the antigen of interest in the clinical sample. In the contrary, negative results indicate no binding of the antigen of the interest to the primary antibodies and thus the absence of the antigen of interest in the clinical sample.

### 1). ELISA: coating first anti-HPV antibody on microtiterplate to detect HPV proteins by a second anti-HPV antibody

To demonstrate a sandwich ELISA on microtiter plate, serum diagnosed with SCC or HPV PCR pos or HPV PCR neg was diluted and used as the analyte for the detection of HPV E6, E7, or L1 protein present in the serum sample. The assay format demonstrated in the ELISA sandwich assay is coating rabbit polyclonal antibody for E6, E7, or L1 protein as the first anti-HPV antibody followed by analyte (serum) and its corresponding second anti-HPV antibody detected by another antibody conjugated with HRP. After incubated with substrate and stopper, OD 450 was taken by a microtiter plate reader. As an example shown in Figure 8, the results demonstrate a ELISA detecting HPV E6, E7 and L1 protein in human serum sample diagnosed with SCC (squamous cell carcinoma) or HPV positive (by PCR) compared to a HPV negative serum (by PCR) using rabbit polyclonal anti-HPV antibody for coating and detection.

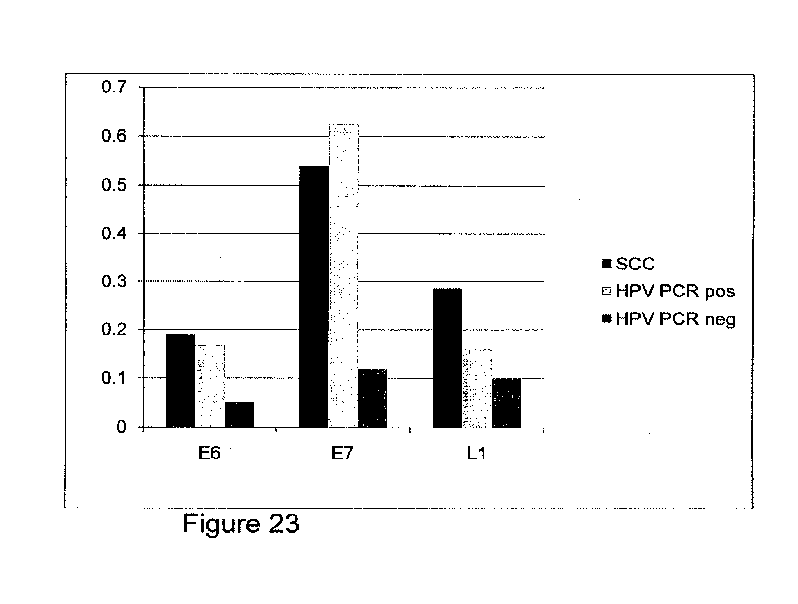

Figure 23 demonstrates the presence of the E6, E7 oncoproteins, and L1 viral proteins can be detected in serum from patients diagnosed with SCC or HPV PCR positive samples using serum from HPV PCR negative samples as a control. The data indicate that E7 is predominate protein detected from serum compared to E6, or L1. Both SCC and HPV PCR pos sample has predominated E6 and E7 protein expression compared to the serum from PCR neg sample. It's noted that SCC has predominant L1 detection compared to HPV PCR neg serum, while HPV PCR pos sample did not have predominant L1 expression compared to HPV PCR neg sample. However, expression of L1 protein is not as predominant as E6, or E7 in either case. These data indicate expression of L1 may be present or absent in the serum depending on the stage and/or cycles of viral infection. However, detection of oncoproteins E6, or E7 in serum from both SCC and HPV PCR pos sample suggests that E6 or E7 represents a better marker for HPV detection in serum. This is the first report for detection of E6, E7 oncoproteins from serum. More serum samples needed to be analyzed.

### 2). Flow Beads assay: coating first anti-HPV antibody on beads to detect HPV proteins by a second anti-HPV antibody

As an example, a first anti-HPV antibody coated on the surface of the beads reacts with HPV proteins in cell lysate of biological sample, forming a complex on the surface of the beads to capture a second anti-HPV antibody. The complex can be detected directly when the second anti-HPV antibody is pre-labeled, or can be detected by adding a pre-labeled antibody capable of binding to the second anti-HPV antibody. The pre-labeled antibody can be labeled with a detection agent including but not limited to, horse radish peroxidase conjugate, biotin, fluorescent, and combinations thereof. As an example, the complex present on the solid surface of beads can be detected by FACS (Fluorescence-activated cell sorting) using anti-mouse or anti-rabbit PE as the secondary antibody. When multiple HPV proteins captured on the beads, multiple second anti-HPV antibodies labeled by different fluorescent dye can be detected simultaneously by the FACS. Thus, the beads assay by FACS provides powerful multiplex assay for detecting one or more HPV proteins from biological samples.

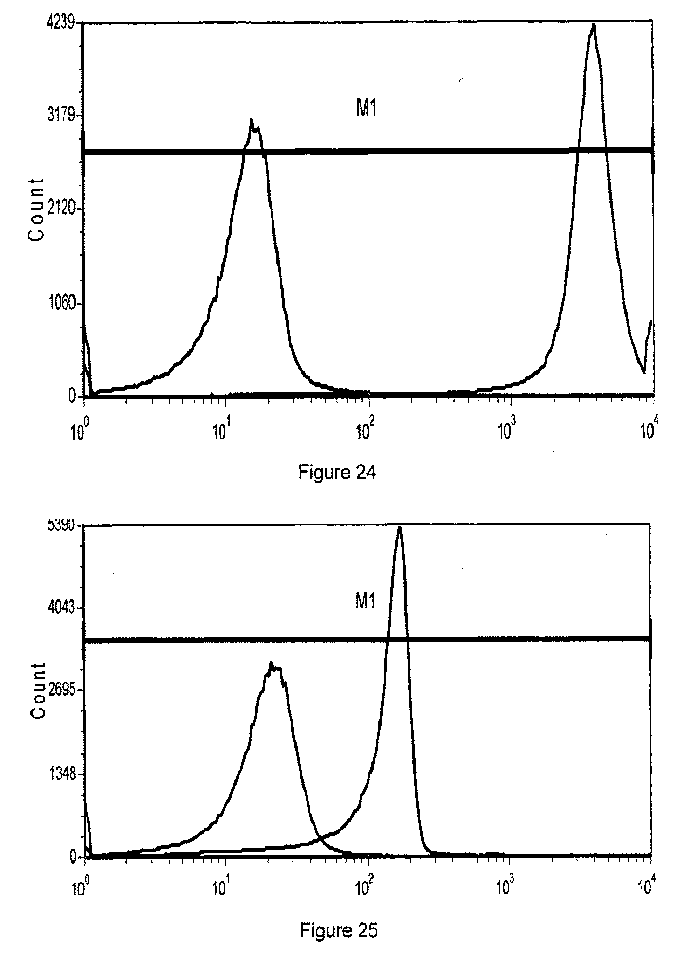

T demonstrate the beads assay described herein can be used for detection of various HPV proteins, various antibody against HPV E6, HPV E7 and HPV L1 were used as coating and detecting antibody to detect HPV E6, HPV E7, and HPV L1 proteins. Figure 24-Figure 27 show results of beads assay detecting HPV 16 L1, HPV 16E6, HPV 18 E6, and HPV 16E7 protein respective, by FACS. As an example, Figure 24 shows results of a sandwich Beads Assay by FACS to detect recombinant HPV16L1 protein using a rabbit polyclonal anti-HPV16 L1 antibody as coating antibody and a mouse monoclonal anti-HPV16 L1 antibody as detecting antibody followed by a secondary antibody, anti-mouse conjugated with PE agent. As data indicated, sample containing purified recombinant HPV 16 L1 protein which is captured on the surface of the beads to be detected by FACS shows discrete peak with higher fluorescent PE (the peak on the right of figure 24) from sample containing buffer as a negative control of the assay (the peak on the left of figure 24). Results indicate about 250 fold of fluorescent difference between the sample containing the specific detecting protein (geometric mean about 2958) and the control sample (geometric mean about 12) with no detecting protein present. Data suggest this beads format provides dynamic range of assays allowed to detect various amount of HPV L1 proteins present in the clinical samples.

As an another example, Figure 25 shows results of a sandwich Beads Assay by FACS to detect recombinant HPV16E6 protein using a rabbit polyclonal anti-HPV16 E6 antibody as coating antibody and a mouse monoclonal anti-HPV16 E6 antibody as detecting antibody followed by a secondary antibody, anti-mouse conjugated with PE agent. As data indicated, sample containing purified recombinant HPV 16 E6 protein which is captured on the surface of the beads to be detected by FACS shows discrete peak with higher fluorescent PE (the peak on the right of figure 25) from sample containing buffer as a negative control of the assay (the peak on the left of figure 25). Results indicate about 7 fold of fluorescent difference between the sample containing the specific detecting protein (geometric mean about 114) and the control sample (geometric mean about 17) with no detecting protein present. These data suggest this beads format provides dynamic range of assays allowed to detect various amount of HPV E6 proteins present in the clinical samples.

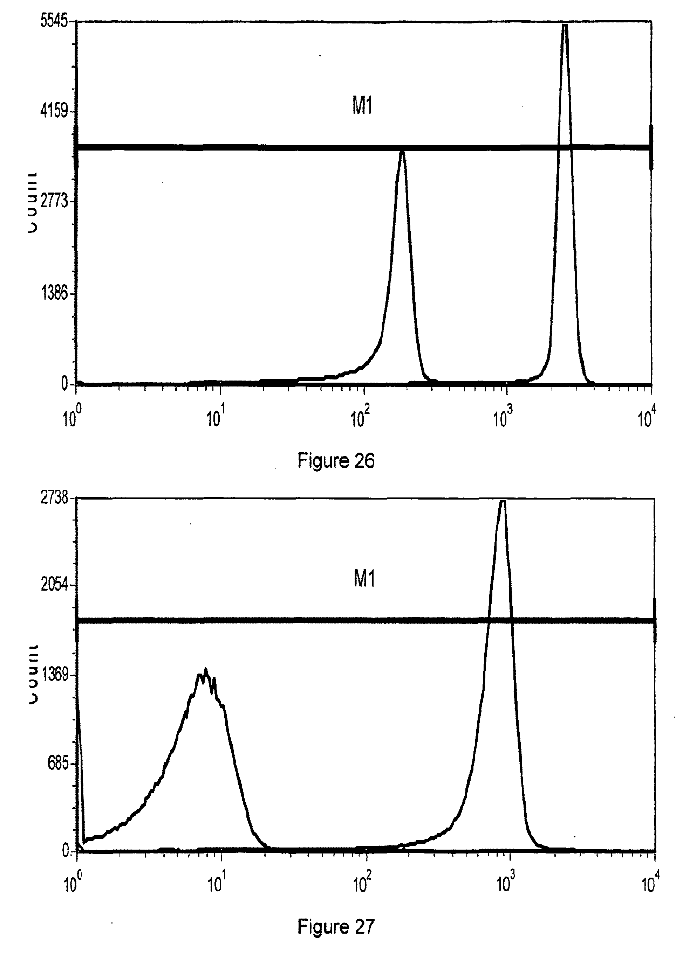

Figure 26 shows results of a sandwich Beads Assay by FACS to detect recombinant HPV16L1 protein using a rabbit polyclonal anti-HPV18 E6 antibody as coating antibody and a mouse monoclonal anti-HPV18 E6 antibody as detecting antibody followed by a secondary antibody, anti-mouse conjugated with PE agent. As data indicated, sample containing purified recombinant HPV 18 E6 protein which is captured on the surface of the beads to be detected by FACS shows discrete peak with higher fluorescent PE (the peak on the right of figure 26) from sample containing buffer as a negative control of the assay (the peak on the left of figure 26). Results indicate about 15 fold of fluorescent difference between the sample containing the specific detecting protein (geometric mean about 2294) and the control sample (geometric mean about 148) with no detecting protein present. These data suggest this beads format provides dynamic range of assays allowed to detect various amount of HPV 18E6 proteins present in the clinical samples.

Figure 27 shows results of a sandwich Beads Assay by FACS to detect recombinant HPV16 E7 protein using a rabbit polyclonal anti-HPV16 E7 antibody as coating antibody and a mouse monoclonal anti-HPV16 E7 antibody as detecting antibody followed by a secondary antibody, anti-mouse conjugated with PE agent. As data indicated, sample containing purified recombinant HPV 16 E7 protein which is captured on the surface of the beads to be detected by FACS shows discrete peak with higher fluorescent PE (the peak on the right of figure 27) from sample containing buffer as a negative control of the assay (the peak on the left of figure 27). Results indicate about 122 fold of fluorescent difference between the sample containing the specific detecting protein (geometric mean about 673) and the control sample (geometric mean about 5.5) with no detecting protein present. These data suggest this beads format provides dynamic range of assays allowed to detect various amount of HPV16 E7 proteins present in the clinical samples.

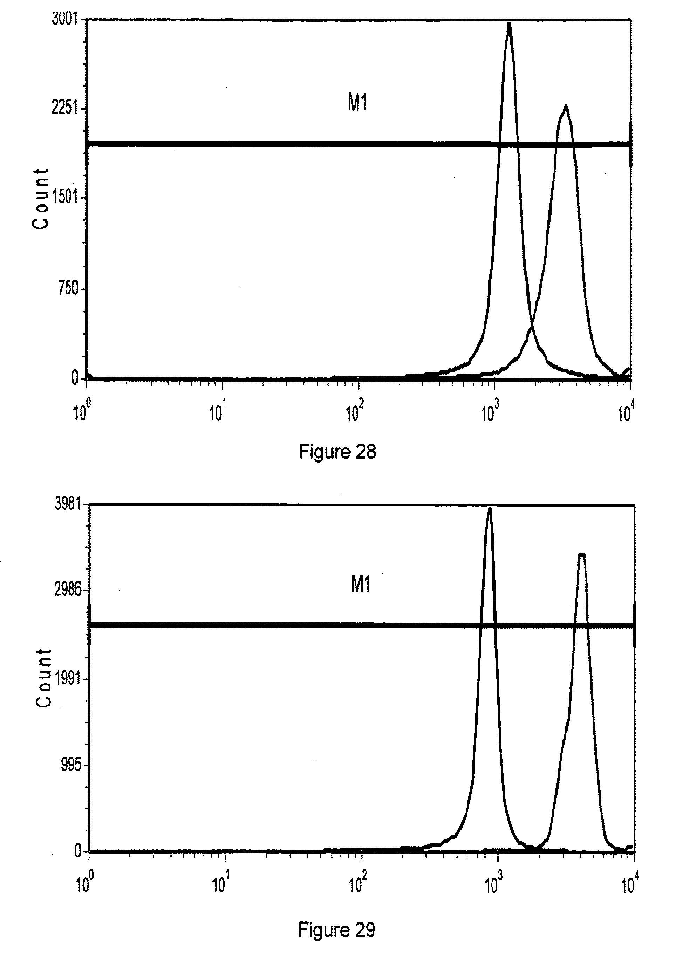

To demonstrate assay performance varies from different assay format by changing the antibody from coating to detecting antibody, Figure 28-Figure 29 show different assay for detecting of HPV 16 E6, and HPV 18 E6 comparing to figure 25, and Figure 26 respectively. As an example, Figure 28 shows results of a sandwich Beads Assay by FACS to detect recombinant HPV16 E6 protein using a rabbit polyclonal anti-HPV16 E6 antibody as detecting antibody and a mouse monoclonal anti-HPV16 L1 antibody as coating antibody followed by a secondary antibody, anti-rabbit conjugated with PE agent. As data indicated, sample containing purified recombinant HPV 16 E6 protein which is captured on the surface of the beads to be detected by FACS shows discrete peak with higher fluorescent PE (the peak on the right of figure 28) from sample containing buffer as a negative control of the assay (the peak on the left of figure 28). Results indicate about only 2 fold of fluorescent difference between the sample containing the specific detecting protein (geometric mean about 2755) and the control sample (geometric mean about 1223) with no detecting protein present. Comparing to Figure 28 to Figure 25 for detection of HPV 16 E6 protein on beads assay, data suggest the beads format shown in Figure 25 provide better dynamic range for assays to allow detecting various amount of HPV E6 proteins present in the clinical samples.

As an another example, Figure 29 shows results of a sandwich Beads Assay by FACS to detect recombinant HPV18E6 protein using a rabbit polyclonal anti-HPV18 E6 antibody as coating antibody and a mouse monoclonal anti-HPV18E6 antibody as detecting antibody followed by a secondary antibody, anti-mouse conjugated with PE agent. As data indicated, sample containing purified recombinant HPV 18E6 protein which is captured on the surface of the beads to be detected by FACS shows discrete peak with higher fluorescent PE (the peak on the right of figure 19) from sample containing buffer as a negative control of the assay (the peak on the left of figure 29). Results indicate about 5 fold of fluorescent difference between the samples containing the specific detecting protein (geometric mean about 3803) and the control sample (geometric mean about 787) with no detecting protein present. Comparing to Figure 29 to Figure 26 for detection of HPV 18 E6 protein on beads assay, data suggest the beads format shown in Figure 26 provides better dynamic range for assays to allow detecting various amount of HPV E6 proteins present in the clinical samples.

3). Rapid flow through assay for detecting HPV infection: The rapid immunological assay can be performed vertically on a membrane or laterally in a strip. The lateral flow-through or diffusion one-step rapid immunological assays may also be referred to as immunochromatographic strip tests would take about 5-15 minutes to obtain results and is easy to use, requiring limited training and does not require instrumentation. The basic principles of the assay include a solid phase nitrocellulose membrane or strip containing the capture agent to react with a swab sample from a Pap smear. If the patient sample contains the target agent, then the capture agent in the nitrocellulose membrane reacts with the target agent, and a complex is formed and migrates in the nitrocellulose membrane through diffusion or capillary action.

The membrane or stick can also be administered to the test human subject during sample collection and/or combined with the cotton swabs, independently or together, to allow the designed immunological reactions to start and thus obtain the test results instantly, for example, right after insertion of a speculum and the swab into the endocervix of the test human subject. Thus, the one-step rapid immunological assay can serve as a primary screening test. The one-step rapid immunological assay can be performed before additional HPV confirmatory tests, including pap smear cytological tests, the immunological assays and nucleic acid hybridization assays as described herein, or combinations thereof.

The vertical rapid immunological test is conducted in a device having a membrane as a capturing/binding surface for coating or spotting a capture agent thereon. The device further contains a pad underneath the membrane to allow the samples and assay reagent to flow through the membrane. Any target proteins, antibodies, or antigens that contained in the samples and specifically interact and bind to the capture agent will not flow through and will be captured and retain on the surface of the membrane, even after several washings to remove non-specific binding. A secondary antibody conjugate with HRP or others can be applied on the surface for detecting any protein-antibody complexes retained on the surface and being visualized by colormetric substrates.

The one-step rapid immunological assay as provided herein is a non-invasive and easy to run assay, similar to the types of over-the-counter pregnancy tests without the need of any particular test instrument. The one-step rapid immunological assay can be an *in vitro* immunochromatographic assay for direct, qualitative detection of common HPV antigens, specific antigens for high risk HPV types, or HPV associated antibodies. The one-step rapid Immunological assay can be used as an adjunct test to Pap smear examination, as point-of-care diagnosis, and/or small clinics or labs. The one-step rapid Immunological assay is suitable for testing condition at room temperature to simply add an obtained sample with or without dilution, wait for a reaction time period for the designed reactions to occur, and visualize the results.

The lateral rapid immunological test is a one-step test using a membrane strip with the capture proteins or antibodies already applied/coated to designated positions on the surface thereof. The only step the test requires is to combine obtained samples having the target proteins or antibodies with a detecting antibody conjugated with collateral gold particles and directly apply the combined mixtures to the membrane strip for the sample fluid to laterally flow through the membrane strip until the designated positions of the surface of the membrane strip. The capture-target-detecting protein-antibody immuno-complexes can be formed and retained on the designated positions where the capture proteins or antibodies are coated. Positive results can be visualized at these designated positions and no washing or separation is required, thus called one-step. The whole procedure for the test takes only minutes, for example, less than 15 minutes, and thus the test is also referred to as a one-step rapid test.

The one-step rapid immunochromatographic assay is a simple, fast, and easy to operate assay, which can be conveniently developed for point-of-care use. In general, there is simply mixing of a sample to be tested with a detection antibody as developed herein, the mixture can be applied onto or is already fixed on a surface (e.g., a membrane or a glass) for a pre-determined reaction time (e.g., in minutes, *etc.*) at optimized incubation temperature, such as at room temperature. The reaction can be optimized to be short for convenience depending on the quality of the detection antibody used and the assay reaction conditions. Thus, a rapid immunological test with short waiting time period can be performed and the assay results is generally designed to be visually scored without the need of any detection instruments.

### A.) One-step HPV lateral flow through assay: stripping first anti-HPV antibody on membrane to detect HPV proteins by a second anti-HPV antibody conjugated with collateral gold particles

The one-step rapid immunological assay may be a membrane or stick test striped with a capture agent, e.g., purified HPV antibodies, recombinant proteins, or HPV-associated antibodies and proteins, *etc.,* as described herein to capture a target agent, e.g., HPV-associated antibodies and HPV-associated proteins, etc., in the clinical sample, followed by an immunoassay detection.

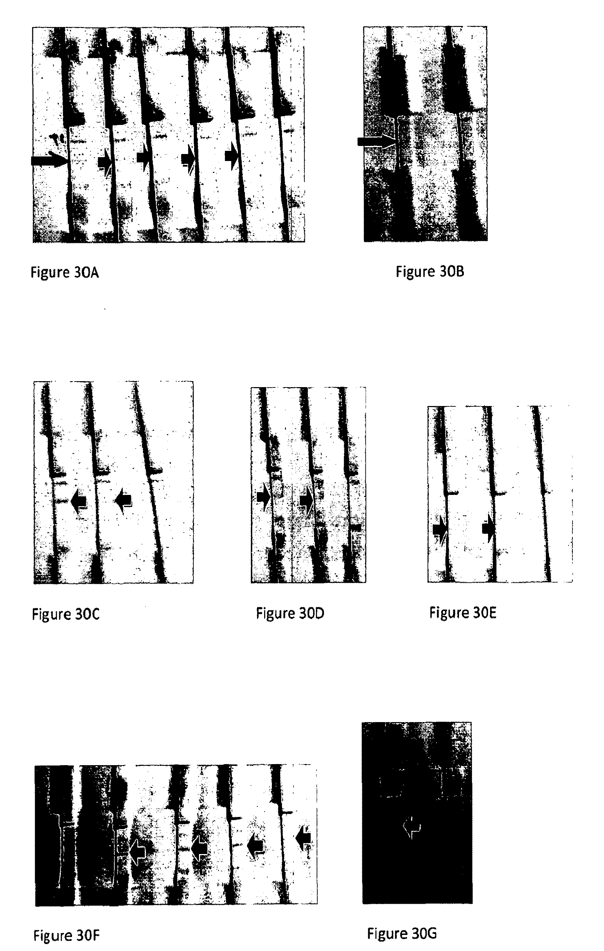

As an example, figures 30A-30G show detection of HPV proteins using antibodies described in this invention on one-step lateral flow through. Figure 30A shows the results of a one-step lateral flow through for detection of HPV L1 recombinant protein using a rabbit anti-L1 polyclonal antibody stripped on membrane, and gold particle conjugated for detection. Test control (TC) shown the line on the top, the second line from the top (arrowed) indicates positive detection of the assay on the left, while the arrow on the right with no visible band shows negative of.the assay. The concentration of the L1 recombinant protein from left to right is 6, 3, 1.5, 0.75, 0.375, 0 ug/ml. These data demonstrate the one-step HPV lateral flow through assay can detect HPV L1 recombinant protein at concentration of 375 ng/ml or lower.

As an example to demonstrate the lateral flow through rapid test can detect HPV L1 protein in clinical samples, Figure 30B show the results of a one-step lateral flow through for detection of HPV L1 protein in serum sample using the same rabbit anti-HPV L1 polyclonal antibody stripped on membrane shown in Figure 30A. Test control (TC) shown the line on the top, the second line from the top (arrowed) indicates positive detection of L1 protein from serum samples of SCC patient (on the left). Serum from a known HPV negative by PCR shown no visible band is used as the negative control of.the assay (on the right). These data demonstrate the one-step HPV lateral flow through assay can detect HPV L1 protein from a SCC serum sample compared to a HPV negative.serum.

To demonstrate detection of HPV E6 proteins using the one-step lateral flow through assay, Figure 30C show the results of a one-step lateral flow through for detection of HPV E6 recombinant protein using a mouse anti-HPV E6 monoclonal antibody stripped on membrane, and gold conjugate for detection. Test control (TC) shown the line on the top, the second line from the top (arrowed) indicates positive detection of the assay on the left, while on the right shown no visible band indicatess the negative control of.the assay. The concentration of the E6 recombinant protein from left to right is 10, 2, 0 ug/ml. Data demonstrate the one-step HPV lateral flow through assay can detect HPV E6 recombinant protein at concentration of 2 ug/ml or lower.

To further demonstrate the lateral flow through device can be used for detecting HPV E6 protein in linical sample, Figure 30D shows the results of a one-step lateral flow through for detection of HPV E6 protein in serum sample using the same mouse anti-E6 monoclonal antibody stripped on membrane shown in Figure 30C. Test control (TC) shown the line on the top, the second line from the top (arrowed) indicates positive detection of E6 protein from SCC serum samples (1^{st} and 2^{nd} from the left). Serum from a known HPV negative by PCR shown no visible band is used as the negative control of the assay (on the right). These data demonstrate the one-step HPV lateral flow through assay can detect HPV E6 protein from a SCC serum sample compared to a HPV negative.serum.

Figure 30E shows the results of a one-step lateral flow through for detection of HPV E6 recombinant protein using another mouse anti-HPV E6 monoclonal antibody stripped on membrane and gold conjugate for detection. Test control (TC) shown the line on the top, the second line from the top (arrowed) indicates positive detection of the assay on the left, while on the right indicated no visible band shows the negative control of.the assay. The concentration of the E6 recombinant protein from left to right is 875, 438, 0 ug/ml. These data demonstrate this one-step HPV lateral flow through assay can detect HPV E6 recombinant protein at concentration of 435 ug/ml or lower.

To demonstrate detection of HPV E7 protein using lateral flow through assay, Figure 30F shows the results of a one-step lateral flow through for detection of HPV E7 recombinant protein using a mouse anti-HPV E7 monoclonal antibody stripped on membrane, and gold conjugated for detection). Test control (TC) shown the line on the top, the second line from the top (arrowed) indicates positive detection of the assay on the right, while on the left shown no visible indicates negative of the assay. The concentration of the E7 recombinant protein from left to right is 0, 660, 66, 6.6, 0.66, ug/ml. These data demonstrate this one-step HPV lateral flow through assay can detect HPV E7 recombinant protein at concentration of 660 ng/ml or lower.

To further demonstrate the lateral flow through device can be used for detecting HPV E7 protein in linical sample, Figure 20G show the results of a one-step lateral flow through for detection of HPV E7 protein in serum sample using the mouse anti-HPV E7 monoclonal antibody stripped on membrane shown in Figure 30F. Test control (TC) shown the line on the top, the second line from the top (arrow) indicates positive detection of E7 protein from serum samples of a known HPV positive by PCR (on the left). Serum from a known HPV negative by PCR shown no visible band is used as the negative control of the assay (on the right). These data demonstrate the one-step HPV lateral flow through assay can detect HPV E7 protein from a known HPV positive serum sample compared to a HPV negative.serum.

The one or more immunological assays using antibodies and purified recombinant proteins derived from HPV early and/or late genes as obtained herein serve as reliable indicators whether HPV infection has occurred. In addition, HPV associated malignancy or pre-malignant cell transformation can be assayed. One of the most useful aspects of the invention is in diagnosing cervical carcinoma, both squamous cell and adenocarcinoma as well as any epithelial cell abnormality associated with oncogenic HPV infection including koilocytosis; hyperkerotosis; precancerous conditions encompasssing intraepithelial neoplasias or intraepithelial lesion; high-grade dysplasias; and invasive or malignant cancers.

Early diagnosis of HPV infection is important for successful prevention and treatment of cervical cancer. Strategies to prevent cervical cancer requires improved HPV testing/screening to cover a broad range of the worldwide population in addition to closely follow-up those subjects with past or present HPV infection and/or precancerous lesions. Importantly, it is known that infection in women for 12-15 years with HPV is required before invasive cancer to develop. It is thus important to be able to assay biomarkers for HPV infection as described herein to pre-screen women early, such that it will be possible to treat HPV infection early and prevent cervical cancer development, rather than having to rely on chemotherapy or radiation to treat cancer malignancy.

### Clauses

1. A method of detecting papillomavirus infection in a human subject, comprising:
   conducting an immunological assay on a clinical sample of the human subject; and staining of a nuclear portion of a human cell from the clinical sample using one or more monoclonal antibodies.
2. The method of claim 1, wherein positive staining of the nuclear portion correlates with the progression to a disease stage selected from the group consisting of an early dysplasia stage, low-grade squamous intracervical lesion (LSIL), high-grade squamous intracervical lesion (HSIL), cervical intraepithelial neoplasm stage 1 , (CIN1), cervical intraepithelial neoplasm stage 2 (CIN2), cervical intraepithelial neoplasm stage 3 (CIN3), and combinations thereof.
3. The method of claim 1 , wherein positive staining of a cytoplasmic portion of the human cell indicates papillomavirus infection being progressed into a disease stage selected from the group consisting of a late dysplasia stage, high-grade squamous intracervical lesion (HSIL), cervical intraepithelial neoplasm (CIN2/3), invasive cervical cancer, squamous cell carcinoma (SCC), adenocarcinoma, and combinations thereof.
4. The method of claim 1, wherein the one or more monoclonal antibodies are generated against one or more purified recombinant papillomavirus proteins.
5. The method of claim 1, wherein the one or more monoclonal antibodies are capable of binding to at least one HPV early viral protein.
6. The method of claim 1, wherein the one or more monoclonal antibodies are capable of binding to at least one HPV late viral protein.
7. The method of claim 1, wherein the one or more monoclonal antibodies are capable of binding to at least one HPV early viral protein and at least one HPV late viral protein.
8. The method of claim 1 , wherein the one or more monoclonal antibodies comprises an antibody capable of binding to the HPV early viral proteins and an antibody capable of binding to the HPV late viral proteins.
9. The method of claim 1, wherein the one or more monoclonal antibodies are selected from the group consisting of anti-HPV E6 monoclonal antibodies, anti-HPV E7 monoclonal antibodies, anti-HPV L1 monoclonal antibodies, and combinations thereof.
10. The method of claim 1, further comprising:
   conducting an immunohistochemistry assay on humans cells from a clinical sample of the human subject using the one or more antibodies generated against one or more purified recombinant papillomavirus proteins; and
   comparing the staining of the nuclear portion with the staining of the cytoplasmic portion of the human cell from the clinical sample.
11. The method of claim 10, wherein more positive staining being observed in the nuclear portion than the cytoplasmic portion of the human cell from the clinical sample indicates papillomavirus infection in the human subject at an early disease stage selected form the group consisting of an early dysplasia stage, low-grade squamous intracervical lesion (LSIL), high-grade squamous intracervical lesion (HSIL), cervical intraepithelial neoplasm 1, intraepithelial neoplasm 2, intraepithelial neoplasm 3 (CIN1, CIN2, CIN3, respectively), and combinations thereof.
12. The method of claim 10, wherein more positive staining being observed in cytoplasmic portion than nuclear portion of the human cell of the clinical sample indicates papillomavirus infection in the human subject at a late disease stage selected from the group consisting of a late dysplasia stage, intraepithelial neoplasm 3 (CIN3), invasive cancer stage, and combinations thereof.
13. The method of claim 1, further comprising:
   conducting an immunohistochemistry assay on a clinical sample of the human subject using two or more antibodies capable of binding to a HPV viral protein selected form the group consisting of E6, E7, L1 proteins, and combinations thereof; and
   comparing the staining of the human cell by the two or more antibodies, wherein positive staining of the human cell by at least one of the two or more antibodies indicates papillomavirus infection in the human subject.
14. The method of claim 1, wherein the immunohistochemistry assay is used to detect HPV infection at various disease stages, the disease stage is selected from the group consisting of early stage HPV infection, late stage HPV infection, early stage cervical cell lesion, late stage cervical cell lesion, low grade of squamous intraepithelial lesion (LSIL), high grade of squamous intraepithelial lesion (HSIL), atypical squamous cells of undetermined significance (ASCUS), cervical intraepithelial neoplasm stage 1, (CIN1), cervical intraepithelial neoplasm stage 2 (CIN2), cervical intraepithelial neoplasm stage 3 (CIN3), developed cervical cancer, adenocarcinoma (ADC), squamous cell carcinoma (SCC), and combinations thereof.
15. The method of claim 1, further comprising:
   providing a clinical sample from the human subject, the clinical sample containing a mixture of morphologically abnormal and normal human cells processed and applied into a thin layer of cells on a slide;
   conducting an immunocytochemistry assay using one or more antibodies generated against one or more purified recombinant papillomavirus proteins, wherein at least one antibody is capable of binding to a papillomavirus early viral protein; and
   staining *in situ* one or more papillomavirus viral proteins from one or more papillomavirus types present in the clinical sample on the slide containing the mixture of morphologically abnormal and normal human cells.
16. The method of claim 1, further comprising
   conducting one or more flow cytometry assays to detect each individual cells by separating each cell from the mixture of the morphologically abnormal and normal human cells; and
   detecting the presence of one or more human papillomavirus viral proteins from one or more papillomavirus types in the mixture of the morphologically abnormal and normal human cells contained in the liquid-based solution from the clinical sample.
17. A method of detecting one or more papillomavirus proteins in a human subject, comprising:
   providing one anti-HPV antibody capable of binding to the one or more papillomavirus proteins from one or more papillomavirus types and present in a clinical sample from the human subject;
   processing the clinical sample into a cell lysate solution containing various proteins including the one or more papillomavirus proteins;
   providing a solid surface having coated thereon at least one protein selected from the group consisting of the anti-HPV antibody and the various proteins present in the cell lysate solution;
   reacting the anti-HPV antibody with the cell lysate solution;
   forming a complex of the one or more papillomavirus proteins with the anti-HPV antibody on the solid surface; and
   detecting the complex on the solid surface to confirm the presence of the one or more papillomavirus proteins present in the clinical sample.
18. The method of claim 17, wherein the one or more papillomavirus proteins are selected from the group consisting of papillomavirus E6 proteins, papillomavirus E7 proteins, papillomavirus L1 proteins, and combinations thereof.
19. The method of claim 17, wherein the solid surface is selected from a group consisting of a surface of a bead, a surface of a strip, a surface of a rapid test strip, a surface of a membrane, a membrane surface of a vertical flow through device, a surface of a microfluidic device, a surface of a blot membrane, a surface of a protein chip, a glass surface, a bottom surface of a microtiter plate, and combinations thereof.
20. The method of claim 19, wherein the complex is detected on the solid surface of one or more beads and the complex is detected by FACS (Fluorescence-activated cell sorting).
21. The method of claim 19, wherein the complex is detected on the solid surface of a strip of a rapid test device with the first anti-HPV antibody immobilized on one end of the strip, and wherein the second anti-HPV antibody and the cell lysate solution are added onto the other end of the strip before flowing laterally on the solid surface of the strip of the rapid test device to form into the complex with first anti-HPV antibody.
22. The method of claim 19, wherein the complex is detected on the solid surface of a membrane of a vertical flow-through rapid test device with the first anti-HPV antibody immobilized thereon, and wherein the cell lysate solution and the second anti-HPV antibody are added sequentially onto the solid surface of the membrane of the vertical flow rapid test device to form into the complex with first anti-HPV antibody.
23. The method of claim 19, wherein the complex is detected on the solid surface of a microfluidic device, and is flowing through the microfluidic device.
24. The method of claim 17, wherein the one or more papillomavirus proteins present in the cell lysate solution are coated on the solid surface before reacting with the anti-HPV antibody.
25. The method of claim 24, wherein the one or more papillomavirus proteins present in the cell lysate solution are coated on the solid surface of a blot membrane.
26. The method of claim 24, wherein the one or more papillomavirus proteins present in the cell lysate solution are coated on the solid surface of a microtiter plate and the anti-HPV antibody is pre-labeled with a detection agent selected from a group consisting of an enzymatic conjugate, biotin, gold particle, and combinations thereof such that the presence of the detection agent indicates the formation of the complex of the one or more papillomavirus proteins and the anti-HPV antibody on the solid surface and the presence of the one or more papillomavirus proteins in the human subject.
27. The method of claim 17, wherein the anti-HPV antibody is coated on the solid surface before reacting with the cell lysate solution having the one or more papillomavirus proteins to detect papillomavirus infection of the human subject.
28. The method of claim 27, wherein the anti-HPV antibody is coated on the solid surface of a protein chip device before reacting with the cell lysate solution.
29. The method of claim 27, wherein the anti-HPV antibody is coated on the solid surface of a microfluidic device before reacting with the cell lysate solution.
30. The method of claim 17, wherein detecting the complex on the solid surface further comprises detecting the presence of a detection agent pre-labeled on one or more proteins selected from a group consisting of the anti-HPV antibody, the various proteins present in the cell lysate solution, a secondary antibody that binds to the anti-HPV antibody, a second anti-HPV antibody different from the anti-HPV antibody and combinations thereof, the second anti-HPV antibody is also capable of binding to the one or more papillomavirus proteins from one or more papillomavirus types and present in the clinical sample.
31. The method of claim 30, wherein the presence of the detection agent is detected by an assay selected from the group consisting of a direct qualitative visualization of the color change of the agent, a readout by an ELISA reader, a readout by a microarray scanner, a readout by a FACS (Fluorescence-activated cell sorting) instrument, and combinations thereof.
32. The method of claim 31, The method of claim 1, further comprising coating an antibody for a cellular protein on the solid surface and comparing the presence of the cellular protein with the presence of the one or more papillomavirus proteins in the clinical sample of the human subject, wherein the cellular protein is selected from the group consisting of p16INK4a, E2F, Ki-67 (MIB-1), MYC protein, CDK4, cyclin-A, cyclin-B, cyclin-E, telomerase-TERC, MCM2 protein, TOP2A protein, heat shock protein 40 (HSP₄₀), heat shock protein 60 (HSP₆₀), heat shock protein 70 (HSP₇₀), CA9/MN protein, laminin 5, laminin proteins, brn-3a, CDK N2 protein, topoisomerase 2A, microsome maintenance proteins-2, microsome maintenance proteins-4, microsome maintenance proteins-5, survivin protein, VEGF protein, p53, RB, p27(kip1), and p21 (waf), and combinations thereof.
33. A lateral flow through device for detecting one or more papillomavirus proteins from one or more papillomavirus types present in a clinical sample being processed into a cell lysate solution, comprising:
   a first solid surface on one end of a strip having a first anti-HPV antibody thereon, the first anti-HPV antibody is capable of binding to the one or more papillomavirus proteins from the one or more papillomavirus types such that the first anti-HPV antibody is able to capture the one or more papillomavirus proteins present in the cell lysate solution onto the solid surface; and
   a second solid surface on the other end of the strip having a second anti-HPV antibody immobilized thereon to react with the cell lysate solution flowing laterally from the first solid surface of the strip to form into a complex with second anti-HPV antibody on the second solid surface of the strip, the second anti-HPV antibody is capable of binding to the one or more papillomavirus proteins such that the second anti-HPV antibody is able to bind and detect the one or more papillomavirus proteins present in the clinical sample.
34. A kit for detecting papillomavirus infection in a human subject, comprising:
   an anti-HPV monoclonal antibody for performing an immunological assays on a clinical sample of the human subject, capable of staining a nuclear portion of one or more human cells from the clinical sample to compare the staining of the nuclear portion with the staining of the cytoplasmic portion of the human cells.
35. A kit for detecting papillomavirus infection in a human subject, comprising:
   an anti-HPV monoclonal antibody, capable of binding to a papillomavirus early protein for conducting an immunocytochemistry assay on a clinical sample from the human subject processed into a solution containing a mixture of morphologically abnormal and normal human cells and applied into a thin layer of cells on a slide for staining *in situ* one or more papillomavirus viral proteins from one or more papillomavirus types present in the clinical sample on the slide containing the mixture of morphologically abnormal and normal human cells.
36. The kit of claim 34 or 35, wherein the anti-HPV antibody is selected from the group consisting of an anti-HPV E7 monoclonal antibody, an anti-HPV E6 monoclonal antibody, a combination of an anti-HPV L1 antibody and anti-HPV E7 monoclonal antibody, a combination of an anti-HPV L1 antibody and anti-HPV E6 monoclonal antibody, a combination of an anti-HPV E6 antibody and anti-HPV E7 monoclonal antibody, and combinations thereof.
37. The kit of claim 34 or 35, wherein the anti-HPV monoclonal antibody is used to detect HPV infection at various disease stages, the disease stage is selected from the group consisting of early stage HPV infection, late stage HPV infection, early stage cervical cell lesion, late stage cervical cell lesion, low grade of squamous intraepithelial lesion (LSIL), high grade of squamous intraepithelial lesion (HSIL), atypical squamous cells of undetermined significance (ASCUS), cervical intraepithelial neoplasm, (CIN1, CIN2, CIN3), developed cervical cancer, adenocarcinoma (ADC), squamous cell carcinoma (SCC), and combinations thereof.

## Claims

1. A method of detecting a papillomavirus protein in a human subject, comprising:
preparing a cell lysate solution using a sample of cells from the human subject, wherein the sample of cells comprises epithelial cells;
coating a portion of a solid surface with a first coat of the cell lysate solution;
reacting the cell lysate solution with an anti-HPV monoclonal antibody, wherein
the anti-HPV monoclonal antibody is capable of binding one or more human papillomavirus viral proteins selected from the group consisting of: HPV E6 proteins, HPV E7 proteins, and a combination thereof, wherein the human papillomavirus viral proteins are associated with a HPV type selected from the group consisting of: HPV6, HPV11, HPV16, HPV18, HPV31, HPV33, HPV39, HPV44, HPV45, HPV51, HPV52, HPV53, HPV58, HPV59, HPV66, HPV68;
forming a complex of the papillomavirus proteins HPV E6 or E7 with the antibody on the portion of the_solid surface;
detecting the complex on the portion of the solid surface, wherein
presence of the complex indicates presence of the papillomavirus protein present in the human subject; and
determining disease stage associated with papillomavirus infection in the human subject based at least in part on the presence of the complex, wherein
the presence of the complex indicates the disease stage as a late dysplasia stage.

2. The method of claim 1, wherein the solid surface is selected from a group that comprises a surface of a bead, a surface of a strip, a surface of a rapid test strip, a surface of a membrane, a membrane surface of a vertical flow through device, a surface of a microfluidic device, a surface of a blot membrane, a surface of a protein chip, a glass surface, and a bottom surface of a microtiter plate.

3. The method of claim 1, wherein
the solid surface comprises a surface of a bead;
the complex is on the portion of the surface of the bead; and
the presence of the complex is detected by using FACS (Fluorescence-activated cell sorting).

4. The method of claims 1, wherein
the solid surface comprises a surface of a rapid test strip, wherein the antibody is immobilized on a first end of the rapid test strip;
a second antibody and the cell lysate solution are added onto a second end of the rapid test strip before flowing laterally on the surface of the rapid test strip; and
the complex of the papillomavirus with the antibody is formed.

5. The method of claim 1, wherein
the solid surface comprises a membrane surface of a vertical flow-through device,
wherein
the antibody is immobilized on the solid surface;
the cell lysate solution and a second antibody are added sequentially onto the solid surface; and
the complex of the papillomavirus with the antibody is formed.

6. The method of claim 1, wherein the solid surface comprises a surface of a microfluidic device.

7. The method of claim 1, wherein the papillomavirus protein in the cell lysate solution is coated on the portion of the solid surface before reacting with the antibody, wherein
the solid surface is selected from a group that comprises a blot membrane and a bottom surface of a microtiter plate.

8. The method of claim 1, wherein the antibody is coated on the solid surface before coating the portion of the solid surface with the first coat of the cell lysate solution, wherein the solid surface is selected from a group that comprises a protein chip and a microfluidic device.

9. The method of claim 1, wherein detecting the complex on the portion of the solid surface further comprises
detecting presence of a detection agent pre-labeled on a molecule that is selected from a group comprising the antibody, the papillomavirus protein in the cell lysate solution, a secondary antibody that binds to the antibody, and a second antibody different from the antibody, wherein
the second antibody specifically binds to the papillomavirus protein from one or more papillomavirus types in the sample of cells from the human subject.

10. The method of claim 9, wherein the presence of the detection agent is detected by an assay, wherein
the assay is selected from a group that comprises a direct qualitative visualization of the color change of the agent, a readout by an ELISA reader, a readout by a microarray scanner, a readout by a fluorescent reader, and a readout by a FACS (Fluorescence-activated cell sorting) instrument.

11. The method of claim 1, further comprising
coating the portion of the solid surface with an antibody for a cellular protein and comparing presence of the cellular protein with the presence of the papillomavirus proteins in the sample of cells from the human subject, wherein the cellular protein is selected from a group that comprises p16INK4a, E2F, Ki-67 (MIB-1), MYC protein, CDK4, cyclin-A, cyclin-B, cyclin-E, telomerase-TERC, MCM2 protein, TOP2A protein, heat shock protein 40 (HSP₄₀), heat shock protein 60 (HSP₆₀), heat shock protein 70 (HSP₇₀), CA9/MN protein, laminin 5, laminin proteins, brn-3a, CDK N2 protein, topoisomerase 2A, microsome maintenance proteins-2, microsome maintenance proteins-4, microsome maintenance proteins-5, survivin protein, VEGF protein, p53, RB, p27(kipl), and p21 (waf).

12. The method of claim 1, wherein
the late dysplasia stage is selected from a group that comprises late stage HPV infection, late stage cervical cell lesion, low grade of squamous intraepithelial lesion (LSIL), high grade of squamous intraepithelial lesion (HSIL). atypical squamous cells of undetermined significance (ASCUS), cervical intraepithelial neoplasm stage 1, 2, 3 (CIN1, CIN2, CIN3), developed cervical cancer, adenocarcinoma, and squamous cell carcinoma (SCC).

13. The method of claim 1, wherein the sample of cells is obtained from a cervical swab or a cervical scrape.

14. The method of claim 1, wherein prior to the action of coating with the first coat, coat the portion of the solid surface with a capturing antibody, wherein
the capturing antibody captures the papillomavirus protein in the cell lysate solution.
